# EUROPÄISCHE PATENTSCHRIFT

(11) **EP 1 078 088 B1**
(45) Veröffentlichungstag und Bekanntmachung des Hinweises auf die Patenterteilung: **26.07.2006**
(21) Anmeldenummer: 99939765.6
(22) Anmeldetag: 12.05.1999
(51) Int. Cl.: C12N 15/82, C07K 14/415, A01H 5/00, C12N 15/29

(54) **TRANSGENE PFLANZEN MIT VERÄNDERTER AKTIVITÄT EINES PLASTIDÄREN ADP/ATP - TRANSLOKATORS**
TRANSGENIC PLANTS WITH A MODIFIED ACTIVITY OF A PLASTIDIAL ADP/ATP TRANSLOCATOR
VEGETAUX TRANSGENIQUES PRESENTANT L'ACTIVITE MODIFIEE D'UN TRANSLOCATEUR D'ADP/ATP PLASTIDIEN

(30) Priorität: 13.05.1998 DE 19821442
(43) Veröffentlichungstag der Anmeldung: 28.02.2001
(73) Patentinhaber: Bayer BioScience GmbH, 14473 Potsdam (DE)
(72) Erfinder: NEUHAUS, Ekkehard, D-49086 Osnabrück (DE); MOEHLMANN, Torsten, D-32257 Bünde (DE); GRAEVE-KAMPFENKEL, Karl-Heinz, D-55246 Mainz-Kostheim (DE); TJADEN, Joachim, D-49074 Osnabrück (DE)
(74) Vertreter: Vossius & Partner
(86) Internationale Anmeldenummer: PCT/EP1999/003292
(87) Internationale Veröffentlichungsnummer: WO 1999/058654

(56) Entgegenhaltungen:
- WO-A-94/10320
- NEUHAUS H E ET AL.: "Characterization of a novel eukaryotic ATP/ADP translocator located in the plastid envelope of Arabidopsis thaliana L." PLANT JOURNAL, Bd. 11, Nr. 1, 1997, Seiten 73-82, XP002126932 ISSN: 0960-7412 in der Anmeldung erwähnt
- MÖHLMANN T ET AL.: "Occurence of two plastidic ATP/ADP transporters in Arabidopsis thaliana L" EUROPEAN JOURNAL OF BIOCHEMISTRY, Bd. 252, Nr. 3, März 1998 (1998-03), Seiten 353-359, XP000865628 ISSN: 0014-2956
- EMMERMANN M ET AL.: "The ADP/ATP translocator from potato has a long amino-terminal extension" CURRENT BIOLOGY, Bd. 20, Nr. 5, November 1991 (1991-11), Seiten 405-410, XP000863257

### SEQUENZPROTOKOLL

(1) ALLGEMEINE ANGABEN:
   (i) ANMELDER:
      (A) NAME: PlantTec Biotechnologie GmbH Forschung & Entwicklung
      (B) STRASSE: Hermannswerder 14
      (C) ORT: Potsdam
      (E) LAND: Deutschland
      (F) POSTLEITZAHL: 14473
   (i) ANMELDER:
      (A) NAME: Max-Planck-Gesellschaft zur Förderung der Wissenschaften e. V.
      (B) STRASSE: keine
      (C) ORT: Berlin
      (E) LAND: Deutschland
      (F) POSTLEITZAHL: keine
   (ii) BEZEICHNUNG DER ERFINDUNG: Transgene Pflanzen mit veränderter Aktivität eines plastidären ADP/ATP-Translokators
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure "
   (iii) HYPOTHETISCH: JA
   (iv) ANTISENSE: NEIN
   (xi) SEQUENZBESCHREIHUNG: SEQ ID NO: 1:
      CGTGAGAGAT AGAGAGCTCG AGGGTCTGAT TCAAACC 37
(2) ANGABEN ZU SEQ ID NO: 2:
   (i) SEQUENZKENNZEICHEN:
      (A) LÄNGE: 26 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: NEIN
   (iv) ANTISENSE: NEIN
   (xi) SEQUENZBESCHREIBUNG: SEQ ID NO: 2:
      GATACAACAG GAATCCTGGA TGAAGC 26
(2) ANGABEN ZU SEQ ID NO: 3:
   (i) SEQUENZKENNZEICHEN:
      (A) LÄNGE: 56 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure "
   (iii) HYPOTHETISCH: JA
   (iv) ANTISENSE: NEIN
   (xi) SEQUENZBESCHREIBUNG: SEQ ID NO: 3:
      GAATTCCTGC AGCCCGGGGG ATCCACTAGT CTCGAGAAGT GGCTGGGGGC CTTTCC 56
(2) ANGABEN ZU SEQ ID NO: 4:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: NEIN
   (iv) ANTISENSE: NEIN
   (xi) SEQUENZBESCHREIBUNG: SEQ ID NO: 4:
      TCTAGAGGCC AAGGCGGCCG CTTCAACGGA CTGCAGTGC 39

## Beschreibung

Die vorliegende Erfindung betrifft transgene Pflanzenzellen und Pflanzen mit einer erhöhten plastidären ADP/ATP-Translokatoraktivität. Derartige Zellen und Pflanzen weisen einen erhöhten Ertrag, vorzugsweise einen erhöhten Öl- und/oder Stärkegehalt auf und synthetisieren vorzugsweise eine Stärke mit erhöhtem Amylosegehalt.
Ferner betrifft die vorliegende Erfindung transgene Pflanzenzellen und Pflanzen mit einer verringerten plastidären ADP/ATP-Translokatoraktivität. Derartige Zellen und Pflanzen synthetisieren eine Stärke mit verringertem Amylosegehalt.

Auf dem Gebiet der Agrar- und Forstwirtschaft ist man stetig darum bemüht, Pflanzen mit erhöhtem Ertrag zur Verfügung zu stellen, insbesondere um die Ernährung der fortwährend anwachsenden Weltbevölkerung sicherzustellen und die Versorgung mit nachwachsenden Rohstoffen zu gewährleisten. Traditionell wird versucht, ertragreiche Pflanzen durch Züchtung zu erhalten. Dieses ist jedoch zeit- und arbeitsintensiv. Ferner müssen entsprechende Züchtungsprogramme für jede interessierende Pflanzenart durchgeführt werden.
Fortschritte wurden zum Teil bereits durch die genetische Manipulation von Pflanzen erzielt, d.h. durch die gezielte Einführung und Expression von rekombinanten Nucleinsäuremolekülen in Pflanzen. Derartige Ansätze haben den Vorteil, daß sie in der Regel nicht auf eine Pflanzenart beschränkt sind, sondern sich auch auf andere Pflanzenarten übertragen lassen.
So wurde beispielsweise in EP-A 0 511 979 beschrieben, daß die Expression einer prokaryontischen Asparagin-Synthetase in Pflanzenzellen unter anderem zu einer erhöhten Biomasseproduktion führt. Die WO 96/21737 beschreibt z.B. die Ertragssteigerung in Pflanzen durch Expression von de- oder unregulierter Fructose-1,6-bisphosphatase, aufgrund der Erhöhung der Photosynthaserate. Trotzdem besteht nach wie vor Bedarf an generell anwendbaren Verfahren zur Verbesserung des Ertrages bei land- oder forstwirtschaftlich interessanten Pflanzen.
Im Hinblick auf die zunehmende Bedeutung, die pflanzlichen Inhaltsstoffen als erneuerbaren Rohstoffquellen in letzter Zeit beigemessen wird, ist ferner eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen. Ferner ist es erforderlich, die Ausbeute an diesen pflanzlichen Inhaltsstoffen zu erhöhen, um die Effizienz der Produktion erneuerbarer Rohstoffquellen aus Pflanzen zu steigern.
Neben Fetten und Proteinen stellen Öle und Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist. Hierbei sind vor allem Kartoffel und Mais interessante Pflanzen, da sie für die Stärkeproduktion wichtige Kulturpflanzen darstellen.
Das Polysaccharid Stärke, das einen der wichtigsten Speicherstoffe im Pflanzenreich darstellt, findet neben der Verwendung im Nahrungsmittelbereich auch eine breite Verwendung als nachwachsender Rohstoff für die Herstellung industrieller Produkte.

Die Stärkeindustrie besitzt ein großes Interesse an Pflanzen mit erhöhtem Stärkegehalt, der in der Regel gleichbedeutend mit einem erhöhtem Trockengewicht ist. Ein erhöhtes Trockengewicht steigert den Wert der in der Stärkeindustrie verarbeiteten Pflanzen (Mais, Kartoffel, Tapioka, Weizen, Gerste, Reis etc.) wegen der höheren Ausbeute an Stärke. Zusätzlich bieten Pflanzenzellen oder pflanzliche Organe, die mehr Stärke enthalten, Vorteile bei der Weiterverarbeitung in der Nahrungsmittelindustrie, weil sie weniger Fett bzw. Frittieröl absorbieren und daher zu "gesünderen" Produkten mit reduziertem Kaloriengehalt führen. Diese Eigenschaft ist z. B. von großem Interesse bei der Herstellung von "Popcorn", "corn flakes" aus Mais oder von Pommes frites, Chips oder Kartoffelpuffer aus Kartoffeln.

Für die Kartoffelstärke verarbeitende Industrie stellt das Trockengewicht (Stärkegehalt) die entscheidende Größe dar, weil es die Prozessierungskosten bestimmt. Ein erhöhtes Trockengewicht (Stärkegehalt) bedeutet bei gleichbleibendem Ertrag, daß der Wassergehalt der Kartoffelknollen verringert ist. Der verringerte Wassergehalt führt zu verringerten Transportkosten und zu einer Reduzierung der beim Kochen erforderlichen Garzeit.
Es erscheint daher wünschenswert, Pflanzenzellen und Pflanzen zur Verfügung zu stellen, die einen erhöhten Stärkegehalt aufweisen, sowie Verfahren zur Herstellung derartiger Pflanzenzellen und Pflanzen.
Ferner erscheint es wünschenswert, Stärken zur Verfügung zu stellen, deren Gehalt an Amylose und Amylopektin den Anforderungen der verarbeitenden Industrie entspricht. Dabei sind sowohl Stärken mit einem erhöhtem Amylosegehalt als auch Stärken mit einem verringerten Amylosegehalt von Interesse, da diese sich jeweils für besondere Verwendungszwecke besonders gut eignen.

Somit liegt der vorliegenden Erfindung die Aufgabe zugrunde, Pflanzenzellen und Pflanzen zur Verfügung zu stellen, welche im Vergleich zu entsprechenden nicht modifizierten Wildtyp-Pflanzenzellen und Wildtyp-Pflanzen einen erhöhten Ertrag vorzugsweise an Öl und/oder Stärke aufweisen und/oder eine Stärke mit einem veränderten Amylosegehalt synthetisieren.

Diese Aufgabe wird durch die Bereitstellung der in den Patentansprüchen bezeichneten Ausführungsformen gelöst.

Somit betrifft die vorliegende Erfindung transgene Pflanzenzellen, die genetisch modifiziert sind, wobei die genetische Modifikation in der Einführung eines fremden Nucleinsäuremoleküls besteht, dessen Vorhandensein oder dessen Expression zur Erhöhung der plastidären ADP/ATP-Translokator-Aktivität in den transgenen Zellen führt im Vergleich zu entsprechenden nicht genetisch modifizierten Pflanzenzellen aus Wildtyp-Pflanzen.

Die genetische Modifikation kann dabei jede genetische Modifikation sein, die zu einer Erhöhung der plastidären ADP/ATP-Translokatoraktivität führt. Möglich ist beispielsweise die sogenannte "in situ-Aktivierung", wobei die genetische Modifikation eine Veränderung der regulatorischen Bereiche endogener ADP/ATP-Translokator-Gene ist, die zu einer verstärkten Expression dieser Gene führt. Erreicht werden kann dies beispielsweise durch die Einführung eines sehr starken Promotors vor die entsprechenden Gene, z. B. durch homologe Rekombination.

Ferner besteht die Möglichkeit, die Methode des sogenannten "activation taggings" anzuwenden (siehe z. B. Walden et al., Plant J. (1991), 281-288; Walden et al., Plant Mol. Biol. 26 (1994), 1521-1528). Diese Methode beruht auf der Aktivierung endogener Promotoren durch "enhancer"-Elemente, wie z. B. dem Enhancer des 35S RNA-Promoters des Blumenkohlmosaikvirus oder dem Octopinsynthase-Enhancers.

In einer bevorzugten Ausführungsform besteht die genetische Modifikation jedoch darin, daß ein fremdes Nucleinsäuremolekül in das Genom der Pflanzenzelle eingeführt wird, welches einen plastidären ADP/ATP-Translokator codiert.

Der Begriff "transgen" bedeutet daher, daß die erfindungsgemäße Pflanzenzelle mindestens ein fremdes Nucleinsäuremolekül stabil in dem Genom integriert enthält, vorzugsweise ein Nucleinsäuremolekül, das einen plastidären ADP/ATP-Translokator codiert.

Unter dem Begriff "fremdes Nucleinsäuremolekül" wird vorzugsweise ein Nucleinsäuremolekül verstanden, das ein Protein mit der biologischen Aktivität eines plastidären ADP/ATP-Translokators codiert und das entweder natürlicherweise in entsprechenden Pflanzenzellen nicht vorkommt, oder das in der konkreten räumlichen Anordnung nicht natürlicherweise in den Pflanzenzellen vorkommt oder das an einem Ort im Genom der Pflanzenzelle lokalisiert ist, an dem es natürlicherweise nicht vorkommt. Bevorzugt ist das fremde Nucleinsäuremolekül ein rekombinantes Molekül, das aus verschiedenen Elementen besteht, dessen Kombination oder spezifische räumliche Anordnung natürlicherweise in pflanzlichen Zellen nicht auftritt. Die erfindungsgemäßen transgenen Pflanzenzellen enthalten mindestens ein fremdes Nucleinsäuremolekül, das ein Protein mit der biologischen Aktivität eines plastidären ADP/ATP-Translokators codiert, wobei dieses vorzugsweise mit regulatorischen DNA-Elementen verknüpft ist, die die Transkription in pflanzlichen Zellen gewährleisten, insbesondere mit einem Promotor.

Prinzipiell kann das fremde Nucleinsäuremolekül jedes beliebige Nucleinsäuremolekül sein, das einen ADP/ATP-Translokator codiert, der nach Expression in der inneren Membran von Plastiden lokalisiert ist. Ein ptastidärer ADP/ATP-Translokator ist dabei ein Protein, das den Transport von ATP in die Plastiden hinein und von ADP aus den Plastiden heraus katalysiert. Bekannt sind solche Nucleinsäuremoleküle beispielsweise aus Arabidopsis thaliana (Kampfenkel et al., FEBS Lett. 374 (1995), 351-355; Genbank Acc. No. X94626 und Acc. No. Z49227) oder aus Kartoffel (Genbank Acc. No. Y10821). Mit Hilfe dieser bekannten Nucleinsäuremoleküle ist es dem Fachmann möglich nach Standardverfahren, beispielsweise durch heterologes Screening, entsprechende Sequenzen aus anderen Organismen, insbesondere pflanzlichen zu isolieren. Es können insbesondere auch nicht-pflanzliche Nucleinsäuremoleküle verwendet werden, die einen ADP/ATP-Translokator codieren und die mit einer targeting-Sequenz verknüpft sind, die die Lokalisierung in der inneren Plastidenmembran gewährleistet. So ist z. B. ein ADP/ATP-Translokator aus Rickettsia prowazekii bekannt (Williamson et al., Gene 80 (1989), 269-278) und aus Chlamydia trachomatis.

In einer bevorzugten Ausführungsform codiert das fremde Nucleinsäuremolekül einen plastidären ADP/ATP-Translokator aus *Arabidopsis thaliana,* insbesondere das in Kampfenkel et al. (1995, a. a. O.) beschriebene Protein AATP1.

Die erfindungsgemäßen Zellen lassen sich von natürlicherweise vorkommenden Pflanzenzellen unter anderem dadurch unterscheiden, daß sie ein fremdes Nucleinsäuremolekül enthalten, das natürlicherweise in diesen Zellen nicht vorkommt oder dadurch, daß ein solches Molekül an einem Ort im Genom der Zelle integriert vorliegt, an dem es sonst nicht vorkommt, d.h. in einer anderen genomischen Umgebung. Ferner lassen sich derartige erfindungsgemäße transgene Pflanzenzellen von natürlicherweise vorkommenden Pflanzenzellen dadurch unterscheiden, daß sie mindestens eine Kopie des fremden Nucleinsäuremoleküls stabil integriert in ihr Genom enthalten, gegebenenfalls zusätzlich zu natürlicherweise in den Zellen vorkommenden Kopien eines solchen Moleküls. Handelt es sich bei dem (den) in die Zellen eingeführten Nucleinsäuremolekül(en) um zusätzliche Kopien zu bereits natürlicherweise in den Zellen vorkommenden Molekülen, so lassen sich die erfindungsgemäßen Pflanzenzellen von natürlicherweise vorkommenden Pflanzenzellen insbesondere dadurch unterscheiden, daß diese zusätzliche(n) Kopie(n) an Orten im Genom lokalisiert ist (sind), an denen sie natürlicherweise nicht vorkommt (vorkommen). Dies läßt sich beispielsweise mit Hilfe einer Southern Blot-Analyse nachprüfen.
Weiterhin lassen sich die erfindungsgemäßen Pflanzenzellen von natürlicherweise vorkommenden Pflanzenzellen vorzugsweise durch mindestens eines der folgenden Merkmale unterscheiden: Ist das eingeführte Nucleinsäuremolekül heterolog in Bezug auf die Pflanzenzelle, so weisen die transgenen Pflanzenzellen Transkripte der eingeführten Nucleinsäuremoleküle auf. Diese lassen sich z. B. durch Northem-Blot-Analyse nachweisen. Vorzugsweise enthalten die erfindungsgemäßen Pflanzenzellen ein Protein, das durch ein eingeführtes Nucleinsäuremolekül codiert wird. Dies kann z. B. durch immunologische Methoden, insbesondere durch eine Western-Blot-Analyse nachgewiesen werden.
Ist das eingeführte Nucleinsäuremolekül homolog in Bezug auf die Pflanzenzelle, können die erfindungsgemäßen Zellen von natürlicherweise auftretenden Zellen beispielsweise aufgrund der zusätzlichen Expression der eingeführten fremden Nucleinsäuremoleküle unterschieden werden. Die transgenen Pflanzenzellen enthalten vorzugsweise mehr Transkripte der fremden Nucleinsäuremoleküle. Dies kann z. B. durch Northern Blot Analyse nachgewiesen werden.
Der Begriff "genetisch modifiziert" bedeutet, daß die Pflanzenzelle durch Einführung eines fremden Nucleinsäuremoleküls in ihrer genetischen Information verändert ist und daß das Vorhandensein oder die Expression des fremden Nucleinsäuremoleküls zu einer phänotypischen Veränderung führt. Phänotypische Veränderung bedeutet dabei vorzugsweise eine meßbare Veränderung einer oder mehrerer Funktionen der Zellen. Beispielsweise zeigen die genetisch modifizierten erfindungsgemäßen Pflanzenzellen aufgrund des Vorhandenseins oder bei Expression des eingeführten Nucleinsäuremoleküls eine Erhöhung der Aktivität eines plastidären ADP/ATP-Translokators.
Der Begriff "Erhöhung der Aktivität" bedeutet dabei im Rahmen der vorliegenden Erfindung eine Erhöhung der Expression eines plastidären ADP/ATP-Translokator-Gens, eine Erhöhung der Menge an plastidären ADP/ATP-Translokator-Protein und/oder eine Erhöhung der Aktivität eines plastidären ADP/ATP-Translokators in den Zellen.
Die Erhöhung der Expression kann beispielsweise bestimmt werden durch Messung der Menge an ADP/ATP-Translokator codierenden Transkripten, z.B. durch Northern-Blot-Analyse. Eine Erhöhung bedeutet dabei vorzugsweise eine Erhöhung der Menge an Transkripten im Vergleich zu entsprechenden nicht genetisch modifizierten Zellen um mindestens 10%, bevorzugt um mindestens 20%, insbesondere um mindestens 50% und besonders bevorzugt um mindestens 75%.
Die Erhöhung der Menge an ADP/ATP-Translokator-Protein kann beispielsweise bestimmt werden durch Western-Blot-Analyse. Eine Erhöhung bedeutet dabei vorzugsweise eine Erhöhung der Menge an ADP/ATP-Translokator-Protein im Vergleich zu entsprechenden nicht genetisch modifizierten Zellen um mindestens 10%, bevorzugt um mindestens 20%, insbesondere um mindestens 50% und besonders bevorzugt um mindestens 75%.

Die Aktivität des plastidären ADP/ATP-Translokators läßt sich beispielsweise bestimmen, indem Plastiden aus entsprechendem Gewebe isoliert werden und die Vₘₐₓ-Werte des ATP-Imports mittels der Silikonölfiltrationsmethode bestimmt werden. Die Reinigung verschiedener Plastidentypen ist z. B. beschrieben in Neuhaus et al. (Biochem J. 296 (1993), 395-401). Die Silikonölfiltrationsmethode ist z. B. beschrieben in Quick et al. (Plant Physiol. 109 (1995), 113-121).

Es wurde überraschenderweise gefunden, daß bei Pflanzen, die derartige Pflanzenzellen mit erhöhter Aktivität des plastidären ADP/ATP-Translokators enthalten, der Ertrag an Inhaltsstoffen und/oder Biomasse zu entsprechenden nicht modifizierten Wildtyp-Pflanzen erhöht ist. Es wurde beispielsweise gefunden, daß in erfindungsgemäßen Pflanzen der Ölgehalt und/oder der Stärkegehalt im Vergleich zu entsprechenden nicht modifizierten Wildtyp-Pflanzen erhöht ist und/oder daß auch der Amylosegehalt dieser Stärken im Vergleich zu entsprechenden nicht modifizierten Wildtyp-Pflanzen erhöht ist.

Der Begriff "Wildtyp-Pflanze" bedeutet dabei, daß es sich um Pflanzen handelt, die als Ausgangsmaterial für die Herstellung der beschriebenen Pflanzen dienten, d.h. deren genetische Information, abgesehen von der eingeführten genetischen Modifikation, der einer erfindungsgemäßen Pflanze entspricht.
Der Begriff "erhöhter Ertrag" bedeutet dabei, daß der Gehalt an Inhaltsstoffen, vorzugsweise an Stärke oder Öl in erfindungsgemäßen Pflanzenzellen um mindestens 10%, bevorzugt um mindestens 20%, besonders bevorzugt um mindestens 30% und insbesondere um mindestens 40% im Vergleich zu Pflanzenzellen von nicht modifizierten Wildtyp-Pflanzen erhöht ist.
Der Begriff "erhöhter Stärkegehalt" bedeutet dabei, daß der Gehalt an Stärke in erfindungsgemäßen Pflanzenzellen um mindestens 10%, bevorzugt um mindestens 20%, besonders bevorzugt um mindestens 30% und insbesondere um mindestens 40% im Vergleich zu Pflanzenzellen von nicht modifizierten Wildtyp-Pflanzen erhöht ist.
Die Bestimmung des Stärkegehalts erfolgt dabei nach den in den angefügten Beispielen beschriebenen Methoden.
Der Begriff "erhöhter Amylosegehalt" bedeutet dabei, daß der Amylosegehalt der in den erfindungsgemäßen Pflanzenzellen synthetisierten Stärke um mindestens 10%, bevorzugt um mindestens 20%, besonders bevorzugt um mindestens 30% und insbesondere um mindestens 40% im Vergleich zu Pflanzenzellen von nicht modifizierten Wildtyp-Pflanzen erhöht ist.
Die Bestimmung des Amylosegehalts erfolgt dabei nach den in den angefügten Beispielen beschriebenen Methoden.

Wie oben erwähnt ist der plastidäre ADP/ATP-Translokator ein Transportprotein, das in der inneren Membran von Plastiden lokalisiert ist (Heldt et al., FEBS Lett. 5 (1969), 11-14; Pozueta-Romero et al., Proc. Nat. Acad. Sci. USA 88 (1991), 5769-5773; Neuhaus, Plant Physiol. 101 (1993) 573-578; Schünemann et al., Plant Physiol. 103 (1993), 131-137) und das den Transport von ATP in die Plastiden hinein und von ADP aus den Plastiden hinaus katalysiert. Somit sorgt der plastidäre ADP/ATP-Translokator für die Versorgung des Stromas mit cytosolischem ATP.
Kampfenkel et al. (FEBS Lett. 374 (1995), 351-355) isolierten erstmals eine cDNA, die einen ADP/ATP-Translokator (AATP1) aus *Arabidopsis thaliana* codiert (Neuhaus et al., Plant J. 11 (1997), 73-82) und die eine große Ähnlichkeit (66.2 % Ähnlichkeit) zum ADP/ATP-Translokator des Gram-negativen Bakteriums Rickettsia prowazekii aufweist. Die AATP1-cDNA aus A. thaliana codiert ein stark hydrophobes Protein bestehend aus 589 Aminosäuren, das 12 potentielle Transmembranhelices aufweist (Kampfenkel et al., FEBS Lett. 374 (1995), 351-355). Diese cDNA konnte in Bäckerhefe und E. coli funktional exprimiert werden. Nach Extraktion des Proteins und Rekonstitution in Proteoliposomen konnte eine Steigerung der ATP-Transportrate festgestellt werden (Neuhaus et al., Plant J. 11 (1997), 73-82). Mit Hilfe von Antikörpern gegen ein Peptidfragment des AATP1 aus A. thaliana konnte gezeigt werden, daß der ADP/ATP-Translokator AATP1 in der inneren Chloroplastenhüllmembran lokalisiert ist (Neuhaus et al., Plant J. 11 (1997), 73-82). Die Funktion des plastidären ADP/ATP-Translokators für den pflanzlichen Stoffwechsel konnte bisher nicht eindeutig geklärt werden. Verschiedene Funktionen wurden in Erwägung gezogen, wie z. B., daß die Versorgung des Stromas mit cytosolischem ATP einen Einfluß auf den Import von Proteinen in den Plastiden hinein, auf die Aminosäurebiosynthese, den Fettsäuremetabolismus oder auf den Stärkemetabolismus haben könnte (Flügge und Hinz, Eur. J. Biochem. 160 (1986), 563-570; Tetlow et al., Planta 194 (1994), 454-460; Hill and Smith, Planta 185 (1991), 91-96; Kleppinger-Sparace et al., Plant Physiol. 98 (1992), 723-727).
Daß eine Steigerung der Aktivität des plastidären ADP/ATP-Translokators zu einer Erhöhung des Stärkegehalts in entsprechenden transgenen Pflanzen führt, war jedoch völlig überraschend. Ebenso überraschend ist es, daß die Steigerung der Aktivität des plastidären ADP/ATP-Translokators Auswirkungen auf die molekulare Zusammensetzung der erzeugten Stärke hat. So weist beispielsweise Stärke aus Knollen erfindungsgemäßer Kartoffelpflanzen einen erhöhten Amylosegehalt auf im Vergleich zu Stärken aus Knollen nicht-transformierter Kartoffelpflanzen.
Bisher wurde angenommen, daß die molekularen Eigenschaften von Stärken ausschließlich durch die Wechselwirkung von stärkesynthetisierenden Enzymen bestimmt werden, wie z.B. der Verzweigungsenzyme (E.C. 2.4.1.18), der Stärkesynthasen (E.C. 2.4.1.21) und der ADP-Glucosepyrophosphorylase (E.C. 2.7.7.27). Daß die Expression eines plastidären Transportproteins einen Einfluß auf die Struktur der Stärke hat, ist dagegen völlig überraschend.

Die erfindungsgemäßen Pflanzenzellen können aus jeder beliebigen Pflanzenspezies stammen, d.h. sowohl aus monokotylen als auch aus dikotylen Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Allgemein bevorzugt sind Pflanzenzellen aus öl- und/oder stärkesynthetisierenden bzw. öl- und/oder stärkespeichernden Pflanzen. Vorzugsweise betrifft die Erfindung somit Pflanzenzellen aus stärkesynthetisierenden bzw. stärkespeichernden Pflanzen, wie z.B. Getreidearten (Roggen, Gerste, Hafer, Weizen, Hirse, Sago etc.), Reis, Erbse, Mais, Markerbse, Maniok, Kartoffel, Raps, Sojabohne, Hanf, Flachs, Sonnenblume, oder Gemüsearten (Tomate, Chicoree, Gurken, Salat etc.). Bevorzugt sind Pflanzenzellen aus Kartoffel, Sonnenblume, Sojabohne, Reis. Besonders bevorzugt sind Pflanzenzellen aus Mais, Weizen, Raps und Reis.

Ferner sind Gegenstand der Erfindung transgene Pflanzen, die die oben beschriebenen transgenen Pflanzenzellen enthalten. Derartige Pflanzen können beispielsweise durch Regeneration aus erfindungsgemäßen Pflanzenzellen erzeugt werden. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, d.h. Pflanzen, die vom Menschen kultiviert werden für Zwecke der Ernährung oder für technische, insbesondere industrielle Zwecke. Dies können öl- und/oder stärkesynthesisierende bzw. öl- und/oder stärkespeichernde Pflanzen sein. Vorzugsweise betrifft die Erfindung Pflanzen, wie z.B. Getreidearten (Roggen, Gerste, Hafer, Weizen, Hirse, Sago etc.), Reis, Erbse, Mais, Markerbse, Maniok, Kartoffel, Raps, Sojabohne, Hanf, Flachs, Sonnenblume oder Gemüsearten (Tomate, Chicoree, Gurken, Salat etc.). Bevorzugt sind Kartoffel, Sonnenblume, Sojabohne, Reis. Besonders bevorzugt sind Mais, Weizen, Raps und Reis.

Wie vorstehend ausgeführt, wurde überraschenderweise gefunden, daß bei stärkespeichernden Pflanzen, die erfindungsgemäße Pflanzenzellen mit erhöhter Aktivität des plastidären ADP/ATP-Translokators enthalten, der Stärkegehalt im Vergleich zu Wildtyp-Pflanzen erhöht ist und/oder daß auch der Amylosgehalt dieser Stärken im Vergleich zu entsprechenden nicht modifizierten Wildtyp-Pflanzen erhöht ist.

In einer bevorzugten Ausführungsform betrifft die vorliegende Erfindung somit auch stärkespeichernde Pflanzen, die die erfindungsgemäßen Pflanzenzellen enthalten und die im Vergleich zu entsprechenden nicht modifizierten Wildtyp-Pflanzen einen erhöhten Stärkegehalt und/oder einen erhöhten Amylosegehalt dieser Stärke im Vergleich zu entsprechenden nicht modifizierten Wildtyp-Pflanzen aufweisen.
Der Begriff "stärkespeichernde Pflanzen" umfaßt alle Pflanzen mit stärkespeichernden Geweben, wie z.B. Mais, Weizen, Reis, Kartoffel, Roggen, Gerste, Hafer. Bevorzugt sind Reis, Gerste und Kartoffel. Besonders bevorzugt sind Mais und Weizen.

Eine Erhöhung des "Ertrags" ("erhöhter Ertrag"), eine Erhöhung des Stärkegehalts ("erhöhter Stärkegehalt"), eine Erhöhung des Amylosegehalts ("erhöhter Amylosegehalt") und der Begriff "Wildtyp-Pflanze" werden dabei wie bereits oben definiert verstanden und werden auch für die folgenden Ausführungsformen der Erfindung in diesem Sinne verwendet. Der Begriff "erhöhter Ertrag" bedeutet dabei vorzugsweise eine Erhöhung der Produktion an Inhaltsstoffen und/oder Biomasse, insbesondere, wenn diese am Frischgewicht pro Pflanze gemessen wird.

Eine derartige Erhöhung des Ertrags bezieht sich vorzugsweise auf emtebare Pflanzenteile wie Samen, Früchte, Speicherwurzeln, Wurzeln Knollen, Blüten, Knospen, Sprosse, Stämme oder Holz.
Die Erhöhung des Ertrags beträgt erfindungsgemäß mindestens 3 %, bezogen auf die Biomasse und/oder Inhaltsstoffe, im Vergleich zu entsprechenden nichttransformierten Pflanzen desselben Genotyps, wenn diese unter denselben Bedingungen kultiviert werden, bevorzugt mindestens 10 %, besonders bevorzugt mindestens 20 % und insbesondere bevorzugt mindestens 30 % oder gar 40% im Vergleich zu Wildtyp-Pflanzen.
Derartige erfindungsgemäße Pflanzen besitzen beispielsweise gegenüber anderen Pflanzen, die Stärken mit erhöhtem Amylosegehalt synthetisieren, wie z.B. den *amylose-extender-* und den *dull*-Mutanten von Mais, den Vorteil, daß sie neben einem erhöhten Amylosegehalt keinen verringerten, sondern sogar einen erhöhten Stärkegehalt aufweisen.

Ferner sind Gegenstand der vorliegenden Erfindung ölspeichernde Pflanzen, die die erfindungsgemäßen Pflanzenzellen enthalten und die im Vergleich zu entsprechenden nicht modifizierten Wildtyp-Pflanzenzellen, vorzugsweise in Zellen ölspeichernder Gewebe, einen erhöhten Ölgehalt aufweisen.
Der Begriff "ölspeichernde Pflanzen" umfaßt alle Pflanzen, die Öl speichern können, wie z.B. Raps, Canola, Sojabohne, Sonnenblume, Mais, Erdnuß, Weizen, Baumwolle, Ölpalmen, Olivenbäume und Avocado. Bevorzugt sind Mais, Weizen und Sojabohne. Besonders bevorzugt sind Raps und Canola.
Der Begriff "erhöhter Ölgehalt" bedeutet dabei, daß der Gehalt an Öl in erfindungsgemäßen Pflanzenzellen um mindestens 10% bevorzugt um mindestens 20%, besonders bevorzugt um mindestens 30% und insbesondere um mindestens 40% im Vergleich zu Pflanzenzellen von nicht-modifizierten Wildtyp-Pflanzen erhöht ist.
Methoden zur Bestimmung des Ölgehalts sind dem Fachmann bekannt und beispielsweise beschrieben bei Matthaeus und Bruehl, GIT Labor-Fachz. 43 (1999), 151-152, 154-155; Matthaeus, Laborpraxis 22 (1998), 52-55. Die Bestimmung des Ölgehalts kann auch durch nichtinvasive Nahinfrarotspektroskopie erfolgen. Es handelt sich hierbei um eine (gängige züchterische) Analysenmethode, die beispielsweise beschrieben wurde bei: Schulz et al., J. Near Infrared Spectrosc. 6 (1998), A125-A130; Starr et al., J. Agric. Sci. 104 (1985), 317-323.

Pflanzen, die eine erhöhte Konzentration an Öl aufweisen sind von großem kommerziellen Interesse. Maispflanzen beispielsweise, deren Körner neben einem hohen Gehalt an Stärke einen erhöhten Gehalt am Nebenprodukt Öl aufweisen, sind für die "wet milling industry" von großem Interesse, weil das Nebenprodukt einen hohen Wert besitzt. Auch die Futtermittelindustrie interessiert sich für Futterpflanzen mit erhöhtem Ölgehalt, weil solche Pflanzen einen erhöhten Nährwert aufweisen. Für die Ölpflanzen-verarbeitende Industrie bedeutet eine Steigerung des Ölgehalts eine Steigerung der Effizienz des Ölextraktionsprozesses.

Die vorliegende Erfindung betrifft auch ein Verfahren zur Herstellung transgener Pflanzen, die einen erhöhten Ertrag im Vergleich zu Wildtyp-Pflanzen aufweisen, wobei
(a) eine pflanzliche Zelle genetisch modifiziert wird durch die Einführung eines fremden Nucleinsäuremoleküls und die genetische Modifikation zur Erhöhung der Aktivität eines plastidären ADP/ATP-Translokators führt; und
(b) aus der Zelle eine Pflanze regeneriert wird; und gegebenenfalls
(c) aus der Pflanze nach (b) weitere Pflanzen erzeugt werden.

Die vorliegende Erfindung betrifft auch ein Verfahren zur Herstellung transgener Pflanzen, die einen erhöhten Stärkegehalt im Vergleich zu Wildtyp-Pflanzen aufweisen und/oder deren Stärke einen erhöhten Amylosegehalt im Vergleich zu entsprechenden Wildtyp-Pflanzen aufweist, wobei
(a) eine pflanzliche Zelle genetisch modifiziert wird durch die Einführung eines fremden Nucleinsäuremoleküls und die genetische Modifikation zur Erhöhung der Aktivität eines plastidären ADP/ATP-Translokators führt; und
(b) aus der Zelle eine Pflanze regeneriert wird; und gegebenenfalls
(c) aus der Pflanze nach (b) weitere Pflanzen erzeugt werden.

Ferner ist Gegenstand der vorliegenden Erfindung ein Verfahren zur Herstellung transgener Pflanzen, die einen erhöhten Ölgehalt im Vergleich zu Wildtyp-Pflanzen aufweisen, wobei
(a) eine pflanzliche Zelle genetisch modifiziert wird durch die Einführung eines fremden Nucleinsäuremoleküls und die genetische Modifikation zur Erhöhung der Aktivität eines plastidären ADP/ATP-Translokators führt; und
(b) aus der Zelle eine Pflanze regeneriert wird; und gegebenenfalls
(c) aus der Pflanze nach (b) weitere Pflanzen erzeugt werden.

Für die laut Schritt (a) in die Pflanzenzelle eingeführte Modifikation gilt dasselbe, was bereits oben im Zusammenhang mit den erfindungsgemäßen Pflanzenzellen und Pflanzen erläutert wurde.
Die Regeneration der Pflanzen gemäß Schritt (b) kann nach dem Fachmann bekannten Methoden erfolgen.
Die Erzeugung weiterer Pflanzen gemäß Schritt (c) der erfindungsgemäßen Verfahren kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt.

Die vorliegende Erfindung betrifft auch die durch das erfindungsgemäße Verfahren erhältlichen Pflanzen.

Die vorliegende Erfindung betrifft auch Vermehrungsmaterial erfindungsgemäßer Pflanzen sowie der gemäß den erfindungsgemäßen Verfahren hergestellten transgenen Pflanzen, das erfindungsgemäße genetisch modifizierte Zellen enthält. Der Begriff Vermehrungsmaterial umfaßt dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen. Anderes Vermehrungsmaterial umfaßt beispielsweise Früchte, Samen, Sämlinge, Protoplasten, Zellkulturen, etc.

Vorzugsweise handelt es sich bei dem Vermehrungsmaterial um Knollen, besonders bevorzugt Samen.

Die vorliegende Erfindung betrifft ferner die Verwendung von Nucleinsäuremolekülen, die einen plastidären ADP/ATP-Translokator codieren, zur Herstellung von transgenen Pflanzen mit einem im Vergleich zum Wildtyp-Pflanzen erhöhten Ertrag.

Die vorliegende Erfindung betrifft ferner die Verwendung von Nucleinsäuremolekülen, die einen plastidären ADP/ATP-Translokator codieren, zur Herstellung von Pflanzen mit einem im Vergleich zu Wildtyp-Pflanzen erhöhten Stärkegehalt in stärkesynthetisierenden und/oder -speichernden Geweben, oder zur Herstellung von Pflanzen, die eine Stärke synthetisieren, die im Vergleich zu Stärke aus Wildtyp-Pflanzen einen erhöhten Amylosegehalt aufweist. Bevorzugt werden dabei die oben im Zusammenhang mit den erfindungsgemäßen Zellen genannten Nucleinsäuremoleküle verwendet.

Die vorliegende Erfindung betrifft ferner die Verwendung von Nucleinsäuremolekülen, die einen plastidären ADP/ATP-Translokator codieren, zur Herstellung von transgenen Pflanzen mit einem Vergleich zum Wildtyp-Pflanzen erhöhten Ölgehalt.

Die vorliegende Erfindung betrifft ferner transgene Pflanzenzellen, die genetisch modifiziert sind, wobei die genetische Modifikation zur Verringerung der Aktivität eines endogen in der Pflanzenzelle vorkommenden plastidären ADP/ATP-Translokators führt im Vergleich zu nicht genetisch modifizierten Pflanzenzellen entsprechender Wildtyp-Pflanzen.

Der Begriff "transgen" bedeutet in diesem Zusammenhang, daß die erfindungsgemäßen Pflanzenzellen aufgrund einer genetischen Modifikation, insbesondere der Einführung eines fremden Nucleinsäuremoleküls in ihrer genetischen Information von entsprechenden nicht genetisch modifizierten Pflanzenzellen abweichen.

Der Begriff "genetisch modifiziert" bedeutet dabei in diesem Zusammenhang, daß die Pflanzenzelle durch Einführung eines fremden Nucleinsäuremoleküls in ihrer genetischen Information verändert ist und daß das Vorhandensein oder die Expression des fremden Nucleinsäuremoleküls zu einer phänotypischen Veränderung führt. Phänotypische Veränderung bedeutet dabei vorzugsweise eine meßbare Veränderung einer oder mehrerer Funktionen der Zellen. Beispielsweise zeigen genetisch modifizierten erfindungsgemäßen Pflanzenzellen eine Verringerung der Aktivität eines plastidären ADP/ATP- Translokators.

Die Herstellung derartiger erfindungsgemäßer Pflanzenzellen mit einer verringerten Aktivität eines ADP/ATP-Translokators kann durch verschiedene, dem Fachmann bekannte Verfahren erzielt werden, z. B. durch solche, die zu einer Inhibierung der Expression endogener Gene führen, die einen plastidären ADP/ATP-Translokator codieren. Hierzu zählen beispielsweise die Expression einer entsprechenden antisense-RNA, die Expression einer sense-RNA zur Erzielung eines Cosuppressionseffektes, die Expression eines entsprechend konstruierten Ribozyms, das spezifisch Transkripte spaltet, die einen ADP/ATP-Translokator codieren, oder die sogenannte "in-vivo Mutagenese".
Vorzugsweise wird zur Reduzierung der Aktivität eines ADP/ATP-Translokators in den erfindungsgemäßen Zellen eine antisense-RNA exprimiert.
Hierzu kann zum einen ein DNA-Molekül verwendet werden, das die gesamte einen ADP/ATP-Translokator codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt zu bewirken. Es können im allgemeinen Sequenzen bis zu einer Mindestlänge von 15 bp, vorzugsweise einer Länge von 100-500 bp, für eine effiziente antisense-Inhibition insbesondere Sequenzen mit einer Länge über 500 bp verwendet werden. In der Regel werden DNA-Moleküle verwendet, die kürzer als 5000 bp, vorzugsweise Sequenzen, die kürzer als 2500 bp sind.
Möglich ist auch die Verwendung von DNA-Sequenzen, die einen hohen Grad an Homologie zu den endogenen in der Pflanzenzelle vorkommenden Sequenzen haben, die einen plastidären ADP/ATP-Translokator codieren. Die minimale Homologie sollte größer als ca. 65 % sein. Die Verwendung von Sequenzen mit Homologien zwischen 95 und 100 % ist zu bevorzugen.

Alternativ kann die Verringerung der ADP/ATP-Translokator-Aktivität in den erfindungsgemäßen Pflanzenzellen auch durch einen Cosuppressionseffekt erfolgen. Das Verfahren ist dem Fachmann bekannt und ist beispielsweise beschrieben in Jorgensen (Trends Biotechnol. 8 (1990), 340-344), Niebel et al. (Curr. Top. Microbiol. Immunol. 197 (1995), 91-103), Flavell et al. (Curr. Top. Microbiol. Immunol. 197 (1995), 43-46), Palaqui und Vaucheret (Plant. Mol. Biol. 29 (1995), 149-159), Vaucheret et al. (Mol. Gen. Genet. 248 (1995), 311-317), de Borne et al. (Mol. Gen. Genet. 243 (1994), 613-621) und anderen Quellen.

Die Expression von Ribozymen zur Verringerung der Aktivität von bestimmten Proteinen in Zellen ist dem Fachmann ebenfalls bekannt und ist beispielsweise beschrieben in EP-B1 0 321 201. Die Expression von Ribozymen in pflanzlichen Zellen wurde z. B. beschrieben in Feyter et al. (Mol. Gen. Genet. 250 (1996), 329-338).

Ferner kann die Verringerung der ADP/ATP-Translokator-Aktivität in den erfindungsgemäßen Pflanzenzellen auch durch die sogenannte "in vivo-Mutagenese" erreicht werden, bei der durch Transformation von Zellen ein hybrides RNA-DNA-Oligonucleotid ("Chimeroplast") in Zellen eingeführt wird (Kipp, P.B. et al., Poster Session beim " 5^{th} International Congress of Plant Molecular Biology, 21.-27. September 1997, Singapore; R. A. Dixon und C.J. Arntzen, Meeting report zu "Metabolic Engineering in Transgenic Plants", Keystone Symposia, Copper Mountain, CO, USA, TIBTECH 15 (1997), 441-447; internationale Patentanmeldung WO 95/15972; Kren et al., Hepatology 25 (1997), 1462-1468; Cole-Strauss et al., Science 273 (1996), 1386-1389).
Ein Teil der DNA-Komponente des RNA-DNA-Oligonucleotids ist homolog zu einer Nucleinsäuresequenz eines endogenen ADP/ATP-Translokators, weist jedoch im Vergleich zur Nucleinsäuresequenz des endogenen ADP/ATP-Translokators eine Mutation auf oder enthält eine heterologe Region, die von den homologen Regionen umschlossen ist.
Durch Basenpaarung der homologen Regionen des RNA-DNA-Oligonucleotids und des endogenen Nucleinsäuremoleküls, gefolgt von homologer Rekombination kann die in der DNA-Komponente des RNA-DNA-Oligonucleotids enthaltene Mutation oder heterologe Region in das Genom einer Pflanzenzelle übertragen werden. Dies führt zu einer Verringerung der Aktivität des plastidären ADP/ATP-Translokators.

Gegenstand der vorliegenden Erfindung sind daher insbesondere auch transgene Pflanzenzellen,
(a) die ein DNA-Molekül enthalten, das zur Synthese einer antisense RNA führen kann, welche eine Verringerung der Expression von endogenen Genen, die einen plastidären ADP/ATP-Translokator codieren, bewirkt; und/oder
(b) die ein DNA-Molekül enthalten, das zur Synthese einer Cosuppressions-RNA führen kann, welche eine Verringerung der Expression von endogenen Genen, die einen ADP/ATP-Translokator codieren, bewirkt;
(c) die ein DNA-Molekül enthalten, das zur Synthese eines Ribozyms führen kann, welches spezifisch Transkripte von endogenen Genen, die einen ADP/ATP-Translokator codieren, spalten kann; und/oder
(d) die aufgrund einer in vivo-Mutagenese eine Mutation oder eine Insertion einer heterologen DNA-Sequenz in mindestens einem endogenen, einen plastidären ADP/ATP-Translokator codierenden Gen aufweisen, wobei die Mutation oder Insertion eine Verringerung der Expression des Gens bewirkt, oder die Synthese eines inaktiven Transportermoleküls.

Der Begriff "Verringerung der Aktivität" bedeutet im Rahmen der vorliegenden Erfindung eine Verringerung der Expression endogener Gene, die einen ADP/ATP-Translokator codieren, eine Verringerung der Menge an ADP/ATP-Translokator-Protein in den Zellen und/ oder eine Verringerung der biologischen Aktivität des ADP/ATP-Translokator-Proteins in den Zellen.
Die Verringerung der Expression kann beispielsweise bestimmt werden durch Messung der Menge an ADP/ATP-Translokator codierenden Transkripten, z.B. durch Northern-Blot-Analyse. Eine Verringerung bedeutet dabei vorzugsweise eine Verringerung der Menge an Transkripten im Vergleich zu entsprechenden nicht genetisch modifizierten Zellen um mindestens 30%, bevorzugt um mindestens 50%, insbesondere um mindestens 70%, besonders bevorzugt um mindestens 85% und ganz besonders bevorzugt um mindestens 95%.
Die Verringerung der Menge an ADP/ATP-Translokator-Protein kann beispielsweise bestimmt werden durch Western-Blot-Analyse. Eine Verringerung bedeutet dabei vorzugsweise eine Verringerung der Menge an ADP/ATP-Translokator-Protein im Vergleich zu entsprechenden nicht genetisch modifizierten Zellen um mindestens 30%, bevorzugt um mindestens 50%, insbesondere um mindestens 70%, besonders bevorzugt um mindestens 85% und ganz besonders bevorzugt um mindestens 95%.

Es wurde überraschenderweise gefunden, daß bei Pflanzenzellen, bei denen die Expression und somit die Aktivität des plastidären ADP/ATP-Translokators verringert ist, der Stärkegehalt im Vergleich zu entsprechenden nicht modifizierten Pflanzenzellen aus Wildtyp-Pflanzen verringert ist und daß auch der Amylosegehalt dieser Stärken im Vergleich zu entsprechenden nicht modifizierten Pflanzenzellen aus Wildtyp-Pflanzen verringert ist. Insbesondere die Tatsache, daß die Stärken der erfindungsgemäßen Pflanzen eine veränderte Zusammensetzung besitzen, ist überraschend, weil bisher angenommen wurde, daß die molekularen Eigenschaften von Stärken ausschließlich durch die Wechselwirkung von stärkesynthetisierenden Enzymen bestimmt werden, wie z.B. der Verzweigungsenzyme (E.C. 2.4.1.18) und der Stärkesynthasen (E.C. 2.4.1.21). Daß die Expression eines plastidären Transportproteins einen Einfluß auf die Struktur der Stärke hat, ist völlig überraschend.
Der Begriff "verringerter Stärkegehalt" bedeutet im Zusammenhang mit der vorliegenden Erfindung, daß der Gehalt an Stärke in erfindungsgemäßen Pflanzenzellen um mindestens 15%, bevorzugt um mindestens 30%, besonders bevorzugt um mindestens 40% und insbesondere um mindestens 50% im Vergleich zu Pflanzenzellen von nicht modifizierten Pflanzen Wildtyp-Pflanzen verringert ist. Die Bestimmung des Stärkegehalts erfolgt dabei nach den in den Beispielen beschriebenen Methoden.
Der Begriff "verringerter Amylosegehalt" bedeutet dabei, daß der Gehalt an Amylose in den erfindungsgemäßen Pflanzenzellen um mindestens 10%, bevorzugt um mindestens 20%, besonders bevorzugt um mindestens 30% und insbesondere um mindestens 40% im Vergleich zu Pflanzenzellen von nicht modifizierten Pflanzen Wildtyp-Pflanzen verringert ist. Die Bestimmung des Amylosegehalts erfolgt dabei nach den in den Beispielen beschriebenen Methoden.
Der Begriff "Wildtyp-Pflanze" ist dabei wie oben definiert zu verstehen.

Die erfindungsgemäßen Pflanzenzellen können aus jeder beliebigen Pflanzenspezies stammen, d.h. sowohl aus monokotylen als auch aus dikotylen Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugsweise betrifft die Erfindung somit Pflanzenzellen aus stärkesynthetisierende bzw. stärkespeichernde Pflanzen, wie z.B. Getreidearten (Roggen, Gerste, Hafer, Weizen, Hirse, Sago etc.), Reis, Erbse, Mais, Markerbse, Maniok, Kartoffel, Tomate, Raps, Sojabohne, Hanf, Flachs, Sonnenblume, Kuherbse oder Arrowroot. Besonders bevorzugt sind Pflanzenzellen aus Kartoffel.

Ferner sind Gegenstand der Erfindung trangene Pflanzen, die die oben beschriebenen transgenen Pflanzenzellen enthalten. Derartige Pflanzen können durch Regeneration aus erfindungsgemäßen Pflanzenzellen erzeugt werden. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, d.h. Pflanzen, die vom Menschen kultiviert werden für Zwecke der Ernährung oder für technische, insbesondere industrielle Zwecke. Vorzugsweise sind dies stärkesynthetisierende bzw. stärkespeichernde Pflanzen, wie z.B. Getreidearten (Roggen, Gerste, Hafer, Weizen, Hirse, Sago etc.), Reis, Erbse, Mais, Markerbse, Maniok, Kartoffel, Tomate, Raps, Sojabohne, Hanf, Flachs, Sonnenblume, Kuherbse oder Arrowroot. Besonders bevorzugt ist Kartoffel.

Derartige erfindungsgemäße Pflanzen synthetisieren eine Stärke die im Vergleich zur Stärke aus entsprechenden Wildtyp-Pflanzen einen verringerten Amylosegehalt aufweist. Die Begriffe "Verringerung des Amylosegehalts" und "Wildtyp-Pflanzen" sind dabei wie oben beschrieben definiert.

Weiterhin betrifft die vorliegende Erfindung auch ein Verfahren zur Herstellung transgener Pflanzen, deren Stärke einen verringerten Amylosegehalt im Vergleich zu Stärke aus entsprechenden Wildtyp-Pflanzen aufweist, wobei
(a) eine pflanzliche Zelle genetisch modifiziert wird durch die Einführung eines fremden Nucleinsäuremoleküls und die genetische Modifikation zur Verringerung der Aktivität eines endogen in pflanzlichen Zellen vorkommenden plastidären ADP/ATP-Translokators führt; und
(b) aus der Zelle gemäß Schritt (a) hergestellten eine Pflanze regeneriert wird; und
(c) aus der gemäß Schritt (b) erzeugten Pflanze gegebenenfalls weitere Pflanzen erzeugt werden.

Für die laut Schritt (a) in die Pflanzenzelle eingeführte Modifikation gilt dasselbe, was bereits oben im Zusammenhang mit den erfindungsgemäßen Pflanzenzellen und Pflanzen erläutert wurde.
Die Regeneration der Pflanzen gemäß Schritt (b) kann nach dem Fachmann bekannten Methoden erfolgen.
Die Erzeugung weiterer Pflanzen gemäß Schritt (c) der erfindungsgemäßen Verfahren kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt.

In einer bevorzugten Ausführungsform wird die erfindungsgemäßen Verfahren zur Erzeugung transgener Kartoffelpflanzen verwendet.

Die vorliegende Erfindung betrifft auch die durch das erfindungsgemäße Verfahren erhältlichen Pflanzen.

Die vorliegende Erfindung betrifft auch Vermehrungsmaterial erfindungsgemäßer Pflanzen sowie der gemäß den erfindungsgemäßen Verfahren hergestellten transgenen Pflanzen, das erfindungsgemäße genetisch modifizierte Zellen enthält. Der Begriff Vermehrungsmaterial umfaßt dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen. Anderes Vermehrungsmaterial umfaßt beispielsweise Früchte, Samen, Sämlinge, Protoplasten, Zellkulturen, etc. Vorzugsweise handelt es sich bei dem Vermehrungsmaterial um Samen und besonders bevorzugt um Knollen.

Ferner betrifft die vorliegende Erfindung die Verwendung von Nucleinsäuremolekülen, die einen plastidären ADP/ATP-Translokator codieren, von deren Komplementen oder von Teilen solcher Moleküle zur Herstellung von Pflanzen, die eine Stärke mit im Vergleich zu Stärke aus Wildtyp-Pflanzen verringerten Amylosegehalt synthetisieren. Hierfür kommen bevorzugt die Nucleinsäuremoleküle in Betracht, die oben im Zusammenhang mit erfindungsgemäßen Pflanzenzellen, die eine erhöhte ADP/ATP-Translokatoraktivität aufweisen, genannt wurden.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobakterium tumefaciens oder Agrobacterium rhizogenes als Transformationsmittel, die Fusion von Protoplasten, die Injektion, die Elektroporation von DNA, die Einbringung der DNA mittels des biolistischen Ansatzes sowie weitere Möglichkeiten.
Die Verwendung der Agrobakterien-vermittelten Transformation von Pflanzenzellen ist intensiv untersucht und ausreichend in EP 120516; Hoekema, In: The Binary Plant Vector System Offsetdrukkerij Kanters B.V., Alblasserdam (1985), Chapter V; Fraley et al., Crit. Rev. Plant Sci. 4, 1-46 und An et al. EMBO J. 4 (1985), 277-287 beschrieben worden. Für die Transformation von Kartoffel, siehe z.B. Rocha-Sosa et al., EMBO J. 8 (1989), 29-33.).
Auch die Transformation monokotyler Pflanzen mittels Agrobakterium basierender Vektoren wurde beschrieben (Chan et al., Plant Mol. Biol. 22 (1993), 491-506; Hiei et al., Plant J. 6 (1994) 271-282; Deng et al, Science in China 33 (1990), 28-34; Wilmink et al., Plant Cell Reports 11 (1992), 76-80; May et al., Bio/Technology 13 (1995), 486-492; Conner und Domisse, Int. J. Plant Sci. 153 (1992), 550-555; Ritchie et al, Transgenic Res. 2 (1993), 252-265). Alternatives System zur Transformation von monokotylen Pflanzen ist die Transformation mittels des biolistischen Ansatzes (Wan und Lemaux, Plant Physiol. 104 (1994), 37-48; Vasil et al., Bio/Technology 11 (1993), 1553-1558; Ritala et al., Plant Mol. Biol. 24 (1994), 317-325; Spencer et al., Theor. Appl. Genet. 79 (1990), 625-631), die Protoplastentransformation, die Elektroporation von partiell permeabilisierten Zellen, die Einbringung von DNA mittels Glasfasern. Insbesondere die Transformation von Mais wird in der Literatur mehrfach beschrieben (vgl. z. B. WO 95/06128, EP 0513849, EO 0465875, EP 292435; Fromm et al., Biotechnology 8 (1990), 833-844; Gordon-Kamm et al., Plant Cell 2 (1990), 603-618; Koziel et al., Biotechnology 11 (1993), 194-200; Moroc et al., Theor. Appl. Genet. 80 (1990), 721-726).
Auch die erfolgreiche Transformation anderer Getreidearten wurde bereits beschrieben, z.B. für Gerste (Wan und Lemaux, s.o.; Ritala et al., s.o.; Krens et al., Nature 296 (1982), 72-74) und für Weizen (Nehra et al., Plant J. 5 (1994), 285-297).

Zur Expression der Nucleinsäuremoleküle, die einen ADP/ATP-Translokator codieren, in sense- oder antisense-Orientierung in pflanzlichen Zellen werden diese vorzugsweise mit regulatorischen DNA-Elementen verknüpft, die die Transkription in pflanzlichen Zellen gewährleisten. Hierzu zählen insbesondere Promotoren. Generell kommt für die Expression jeder in pflanzlichen Zellen aktive Promotor in Frage.
Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. Sowohl in Bezug auf die Pflanze als auch in Bezug auf das Nucleinsäuremolekül kann der Promotor homolog oder heterolog sein.
Geeignete Promotoren sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endospermspezifische Expression der HMG-Promotor aus Weizen, der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218) oder Shrunken-1 Promotor (Werr et al., EMBO J. 4 (1985), 1373-1380). Es können jedoch auch Promotoren verwendet werden, die nur zu einem durch äußere Einflüsse determinierten Zeitpunkt aktiviert werden (siehe beispielsweise WO 9307279). Von besonderem Interesse können hierbei Promotoren von heat-shock Proteinen sein, die eine einfache Induktion erlauben. Ferner können samenspezifische Promotoren verwendet werden, wie z.B. der USP-Promoter aus Vicia faba, der eine samenspezifische Expression in Vicia faba und anderen Pflanzen gewährleistet (Fiedler et al., Plant Mol. Biol. 22 (1993), 669-679; Bäumlein et al., Mol. Gen. Genet. 225 (1991), 459-467).

Die vorgenannten Ausführungsformen mit den endospezifischen Promotoren sind insbesondere zur Steigerung des Stärkegehalts im Endosperm geeignet. Im Gegensatz dazu ist die Verwendung embryospezifischer Promotoren insbesondere zur Steigerung des Ölgehalts von Interesse, weil in der Regel Öl vorwiegend im Embryo gespeichert wird.

Daher wird gemäß vorliegender Erfindung auch vorzugsweise ein Promotor verwendet, der die Expression im Embryo bzw. im Samen gewährleistet. In einer bevorzugten Ausführungsform der Erfindung ist der Promotor der Globulin-1 (glb1)-Promotor aus Mais (Styer and Cantliffe, Plant Physiol. 76 (1984), 196-200). In einer weiteren Ausführungsform der Erfindung ist der embryospezifische Promotor aus Pflanzen, vorzugsweise aus *Cuphea lanceolata, Brassica rapa* oder *Brassica napus.* Besonders bevorzugt sind die Promotoren pClFatB3 und pClFatB4 (WO 95/07357). Es handelt sich hierbei um Promotoren der Gene ClFatB3 bzw. ClFatB4, die in transgenem Raps bereits erfolgreich zur Biosynthese mittelkettiger Fettsäuren eingesetzt wurden, und daher ein geeignetes Expressionsfenster zur Lösung des vorliegenden Problems besitzen.
In einer weiteren bevorzugten Ausführungsform wird der pCIGPDH-Promotor (WO 95/06733), der Napin- (beispielsweise beschrieben bei Kridl, Seed Sci. Res. 1 (1991), 209-219; Ellerstrom et al., Plant Mol. Biol. 32 (1996), 1019-1027; Stalberg et al., Planta 199 (1996), 515-519) oder der Oleosin-Promotor (beispielsweise beschrieben bei Keddie, Plant Mol. Biol. 24 (1994), 327-340; Plant et al., Plant Mol. Biol. 25 (1994), 193-205) verwendet.

Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z.B. Gielen et al., EMBO J. 8 (1989), 23-29) und sind beliebig austauschbar.

Die erfindungsgemäßen transgenen Pflanzenzellen und Pflanzen synthetisieren vorzugsweise aufgrund der Erhöhung oder Verringerung der Aktivität eines plastidären ADP/ATP-Translokators, eine Stärke, die in ihren physikalischchemischen Eigenschaften, insbesondere dem Amylose/Amylopektin-Verhältnis im Vergleich zu in Wildtyp-Pflanzen synthetisierter Stärke verändert ist. Insbesondere kann eine solche Stärke im Hinblick auf die Viskosität und/oder die Gelbildungseigenschaften von Kleistern dieser Stärke im Vergleich zu Wildtypstärke verändert sein.

Die vorliegende Erfindung betrifft daher ferner Verfahren zur Herstellung einer modifizierten Stärke umfassend den Schritt der Extraktion der Stärke aus einer oben beschriebenen erfindungsgemäßen Pflanze und/oder aus stärkespeichernden Teilen einer solchen Pflanze. Vorzugsweise umfaßt ein solches Verfahren auch den Schritt des Emtens der kultivierten Pflanzen und/oder stärkespeichernder Teile dieser Pflanzen vor der Extraktion der Stärke und besonders bevorzugt ferner den Schritt der Kultivierung erfindungsgemäßer Pflanzen vor dem Ernten. Verfahren zur Extraktion der Stärke von Pflanzen oder von stärkespeichernden Teilen von Pflanzen sind dem Fachmann bekannt. Weiterhin sind Verfahren zur Extraktion der Stärke aus verschiedenen anderen stärkespeichernden Pflanzen beschrieben, z. B. in "Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum-Stärken: Herstellung; von Watson; Kapitel XIII, Seite 469-479: Tapioca-, Arrowroot- und Sagostärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996) 54-57, die Extraktion von Maisstärke im industriellen Maßstab wird in der Regel durch das sogenannte "wet milling" erreicht.)). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.
Gegenstand der vorliegenden Erfindung ist ferner Stärke, die aus den erfindungsgemäßen transgenen Pflanzenzellen, Pflanzen sowie Vermehrungsmaterial erhältlich ist, und Stärke, die durch das oben beschriebene erfindungsgemäße Verfahren erhältlich ist.

Die erfindungsgemäßen Stärken können nach dem Fachmann bekannten Verfahren modifiziert werden und eignen sich in unmodifizierter oder modifizierter Form für verschiedene Verwendungen im Nahrungsmittel- oder Nicht-Nahrungsmittelbereich. Grundsätzlich läßt sich die Einsatzmöglichkeit der Stärke in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoff für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein. Gegenwärtig verläuft es im wesentlichen enzymatisch unter Verwendung von Amyloglucosidase. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch geringeren Verzweigungsgrad oder eine sterische Struktur, die die Zugänglichkeit für die eingesetzten Enzyme begrenzt, könnte dies bewirken.
Der andere Bereich, in dem die Stärke wegen ihrer polymeren Struktur als sogenannte native Stärke verwendet wird, gliedert sich in zwei weitere Einsatzgebiete:
1. Nahrungsmittelindustrie
   Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.
2. Nicht-Nahrungmittelindustrie
   In diesem großen Bereich kann die Stärke als Hilfsstoff für unterschiedliche Herstellungsprozesse bzw. als Zusatzstoff in technischen Produkten eingesetzt. Bei der Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.
2.1 Papier- und Pappeindustrie
   Innerhalb des Papierherstellungsprozesses sind vier Anwendungsbereiche, nämlich Oberfläche, Strich, Masse und Sprühen, zu unterscheiden.
   Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.
2.2 Klebstoffindustrie
   Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.
2.3 Textil- und Textilpflegemittelindustrie
   Ein großes Einsatzfeld für die Stärken als Hilfsmittel und Zusatzstoff ist der Bereich Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechtemden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.
2.4 Baustoffindustrie
   Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.
2.5 Bodenstabilisation
   Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindemden Wirkung den bisher eingesetzten Produkten gleichzusetzen, liegen preislich aber deutlich unter diesen.
2.6 Einsatz bei Pflanzenschutz- und Düngemitteln
   Ein Einsatzbereich liegt bei der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.
2.7 Pharmaka, Medizin und Kosmetikindustrie
   Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbieren und nach kurzer Zeit soweit quellen, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.
2.8 Stärkezusatz zu Kohlen und Briketts
   Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.
2.9 Erz- und Kohleschlammaufbereitung
   Die Stärke kann ferner bei der Erz- und Kohleschlammaufbereitung als Flockungsmittel eingesetzt werden.
2.10 Gießereihilfsstoff
   Ein weiterer Einsatzbereich besteht als Zusatz zu Gießereihilfsstoffen. Bei verschiedenen Gußverfahren werden Kerne benötigt, die aus Bindemittelversetzten Sänden hergestellt werden. Als Bindemittel wird heute überwiegend Bentonit eingesetzt, das mit modifizierten Stärken, meist Quellstärken, versetzt ist.
   Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.
2.11 Einsatz in der Kautschukindustrie
   In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.
2.12 Herstellung von Lederersatzstoffen
   Eine weitere Absatzmöglichkeit der modifizierten Stärken besteht bei der Herstellung von Lederersatzstoffen.
2.13 Stärke in synthetischen Polymeren
   Auf dem Kunststoffsektor zeichnen sich folgende Einsatzgebiete ab: die Einbindung von Stärkefolgeprodukten in den Verarbeitungsprozeß (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthetische Polymer durch Koexpression im Verhältnis von 1 : 1 zu einem 'master batch' kombiniert, aus dem mit granuliertem Polyethylen unter Anwendung herkömmlicher Verfahrenstechniken diverse Produkte hergestellt werden. Durch die Einbindung von Stärke in Polyethylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z.B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amytopektinverhältnis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und -transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.
Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderte Stärken weiteren chemischen Modifikationen unterworfen werden, was zu weiteren Verbesserungen der Qualität für bestimmte der oben beschriebenen Einsatzgebiete führt. Diese chemischen Modifikationen sind grundsätzlich bekannt. Insbesondere handelt es sich dabei um Modifikationen durch
- Hitzebehandlung,
- Säurebehandlung,
- Oxidation und
- Veresterungen,
welche zur Entstehung von Phosphat-, Nitrat-, Sulfat-, Xanthat-, Acetat- und Citratstärken führen. Weitere organische Säuren können ebenfalls zur Veresterung eingesetzt werden:
- Erzeugung von Stärkeethern Stärke-Alkylether, O-Allylether, Hydroxylalkylether, O-Carboxylmethylether, N-haltige Stärkeether, P-haltige Stärkeether, S-haltige Stärkeether
- Erzeugung von vernetzten Stärken
- Erzeugung von Stärke-Pfropf-Polymerisaten

- **Figur 1**: zeigt schematisch das Plasmid pJT31 (AATP1 (*Arabidopsis thaliana*) sense);
- **Figur 2**: zeigt schematisch das Plasmid pJT32 (AATP1 (*Solanum tuberosum*) antisense);

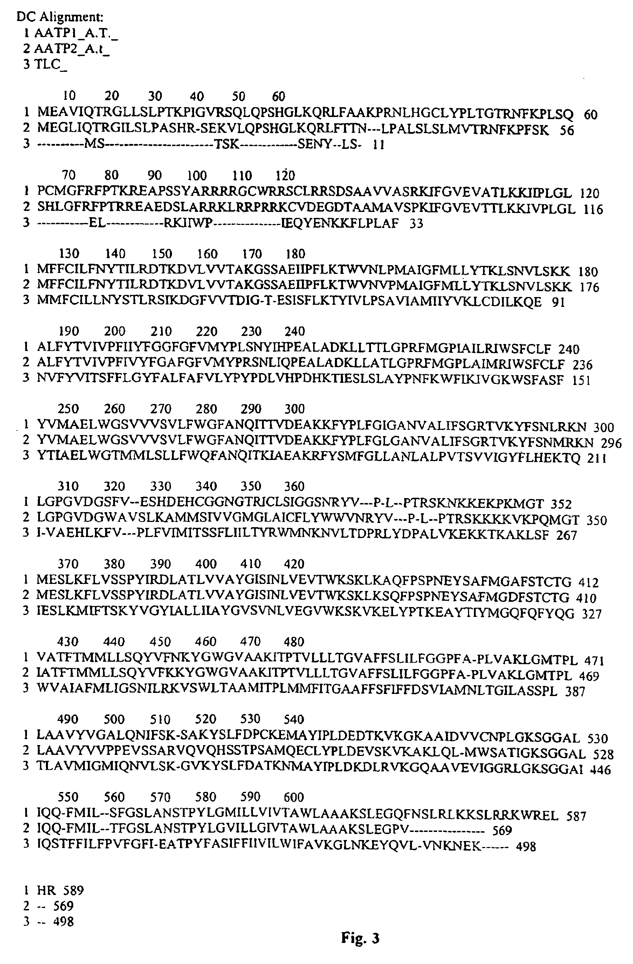

- **Figur 3**: zeigt den Aminosäuresequenzvergleich des AATP2 aus *Arabidopsis thaliana* mit dem AATP1 (A. thaliana) und einem homologen Protein aus Rickettsia prowazekii (Williamson et al., Gene 80 (1989), 269-278);

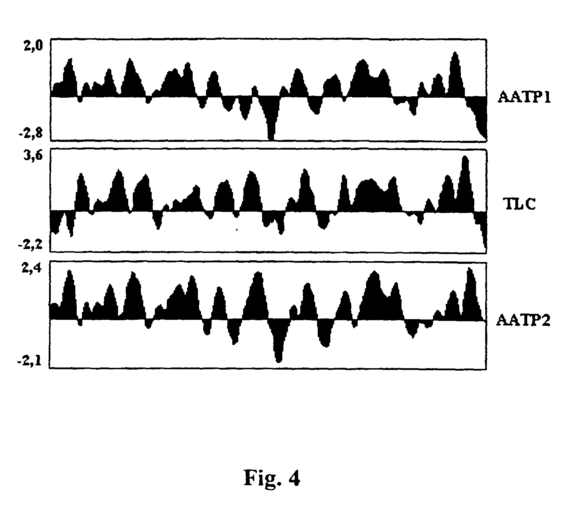

- **Figur 4**: Hydropathie-Analyse von AATP2 (A. thaliana), AATP1 (A. thaliana) und dem Rickettsia ADP/ATP-Translokator nach der Methode von von Heijne et al. (Eur. J. Biochem. 180 (1989), 535-545.) durchgeführt
- **Figur 5**: zeigt eine Northern Blot Analyse der Expression des AATP1 (*Solanum tuberosum*) in Blatt und Knolle von ADP/ATP-Translokator-antisense-Pflanzen.
- **Figur 6**: zeigt eine Northern Blot Analyse der Expression des AATP1 (*Arabidopsis thaliana*) in Blatt und Knolle von ADP/ATP-Translokator-Überexpressionspflanzen.

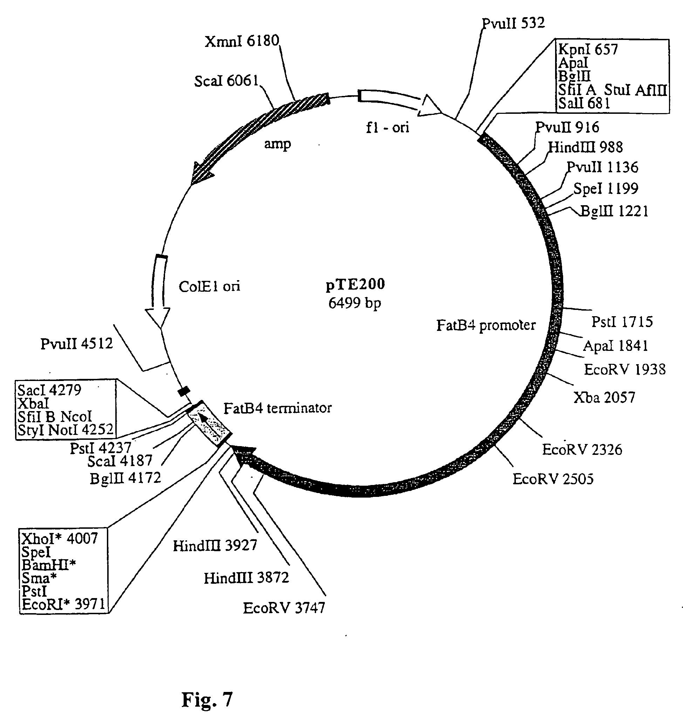

- **Figur 7**: Schematische Karte der Kassette zur embryospezifischen Genexpression pTE200. EcoRI, Smal, BamHI, Xhol, Notl, Xbal, Sacl, Kpnl, Apal, Sall und Sfil markieren Erkennungsstellen für Restriktionsendonukleasen. Aus praktischen Gründen unterscheiden sich Sfil (A) und Sfil (B) in der variablen Nukleotidfolge innerhalb der Erkennungssequenz. Die Abkürzungen kodieren wie folgt: P*ClFatB4* = CIFatB4-Promotor, t*ClFatB4* = ClFatB4-Terminator, amp = bakterielle Resistenz gegenüber Ampicillin, ColE1 ori = "origin of replication" aus dem Plasmid ColE1, f1(-) ori = "origin of replication" aus dem Phagen f1.
- **Figur 8**: Schematische Karte der ADPIATP-Translokatorexpressionskassette pTE208: dieses Derivat des Vektors pTE200 (Figur 7) trägt eine cDNA codierend für einen plastidären ADP/ATP-Translokator aus *Solanum tuberosum* in sense -Orientierung.

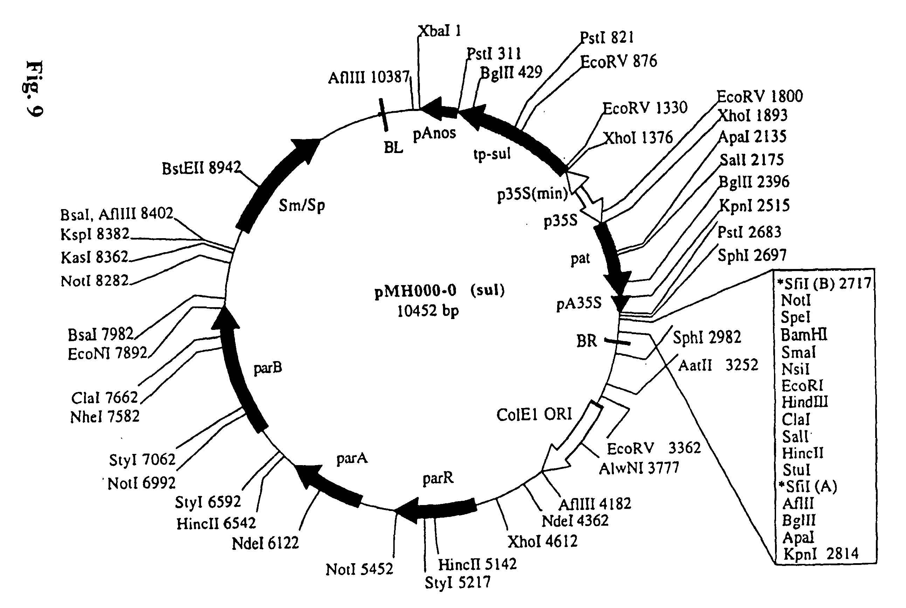

- **Figur 9**: Schematische Karte des binären Vektors pMH000-0. Sfil, Sall, Clal, HindIII, EcoRI, NsiI, SmaI, BamHI, SpeI, NotI, KpnI, BgIII, ApaI, XhoI, Xbal und BstEII markieren Erkennungsstellen für Restriktionsendonukleasen. Sfil (A) und Sfil (B) unterscheiden sich in der variablen Nukleotidfolge ihrer Erkennungssequenz wie angegeben. Dadurch wird nach Sfil-Spaltung eine Rezirkularisierung des Aussgangsplasmids unterbunden und eine gerichtete Insertion der Expressionskassette aus dem pTE200-Derivat möglich. Die Abkürzungen kodieren wie folgt: RB, LB = rechte und linke Grenzregion, t35S - Terminationssignal des 35S *rna* Gens aus CaMV, pat = Phosphinotricinacetyltransferasegen, p35S = Promotor des 35S *rna* Gens aus CaMV, p35S(min) = Minimalpromotor des 35S rna Gens aus CaMV, tp-*sul* = Sulfonamidresistenzgen mit Transitpeptide, t*nos* =Terminationssignal des Nopalinsynthasegens, Sm/Sp = bakterielle Resistenz gegenüber Streptomycin und Spectinomycin, parA, parB und parR = Plasmidvermehrungsfunktionen aus dem Plasmid pVS1 mit großem Wirtsbereich u. a. für *Agrobacterium tumefaciens* und *Escherichia coli.*

Die folgenden Beispiele erläutern die Erfindung.

### Beispiel 1

### Konstruktion des bakteriellen Expressionsvektors pJT118 und Transformation von E. coli

Das AATP2-Protein (Genbank X94626) aus *Arabidopsis thaliana* wurde N-terminal mit einem "Histidin-tag" umfassend 10 Aminosäuren fusioniert.
Hierfür wurde die cDNA, die das gesamte AATP2-Protein aus *Arabidopsis thaliana* codiert, mit Hilfe eines PCR-Ansatzes isoliert. Als sense-Primer diente folgendes Oligonucleotid, welches zusätzlich eine Xhol-Restriktionsschnittstelle trug: cgtgagagatagagagctcgagggtctgattcaaacc (SEQ ID NO: 1); umfaßt die Basenpaare 66-102).
Als antisense-Primer diente ein Oligonucleotid, welches eine zusätzliche BamHl-Restriktionsschnittstelle trug: gatacaacaggaatcctggatgaagc (SEQ ID NO: 2); umfaßt die Basenpaare 1863-1835). Das erhaltene PCR-Produkt wurde über ein Agarosegel gereinigt, mit den Restriktionsenzymen Xhol/BamHl geschnitten und "in frame" in das Plasmid pET16b (Novagene, Heidelberg, Germany) eingeführt. Dies führte dazu, daß die cDNA, die das gesamte AATP2-Protein aus *Arabidopsis thaliana* codiert, am N-Terminus einen Histidine-tag aus 10 Aminosäuren aufweist (His-AATP2). Dieser Vektor wurde pJT118 genannt. Die Sequenz des PCR-Produktes wurde durch Sequenzierung beider Nucleotidstränge überprüft (Eurogentec). Die Transformation von E. coli C43 (Miroux and Walker, J. Mol. Biol. 260 (1996), 289-298) erfolgte nach Standardmethoden.
Der Stamm E. coli C43 erlaubt die heterologe Expression von tierischen (Miroux and Walker, a.a.O.) und pflanzlichen (Tjaden et al., J. Biol. Chem. (1998) (in press)) Membranproteinen.
Nach Transformation dieses Stammes mit dem Vektor pJT118 wurden Aufnahmestudien mit radioaktiv markiertem ADP und ATP durchgeführt. Mit Hilfe dieser Studien konnte gezeigt werden, daß His-AATP2 in E. coli C43 funktional in der Cytoplasmamembran von E. coli exprimiert werden kann. Hierdurch konnte gezeigt werden, daß AATP2 tatsächlich einen ADP/ATP-Translokator codiert. Das Vorhandensein eines N-terminalen Histidine-tags führt zu einer Erhöhung (2x-3x) der Transportaktivität des AATP2 aus A. thaliana in E. coli im Vergleich zum AATP2 ohne N-terminalen His-tag.

### Beispiel 2

### Konstruktion des Plasmids pJT31 und Einführung des Plasmids in das Genom von Kartoffelpflanzen

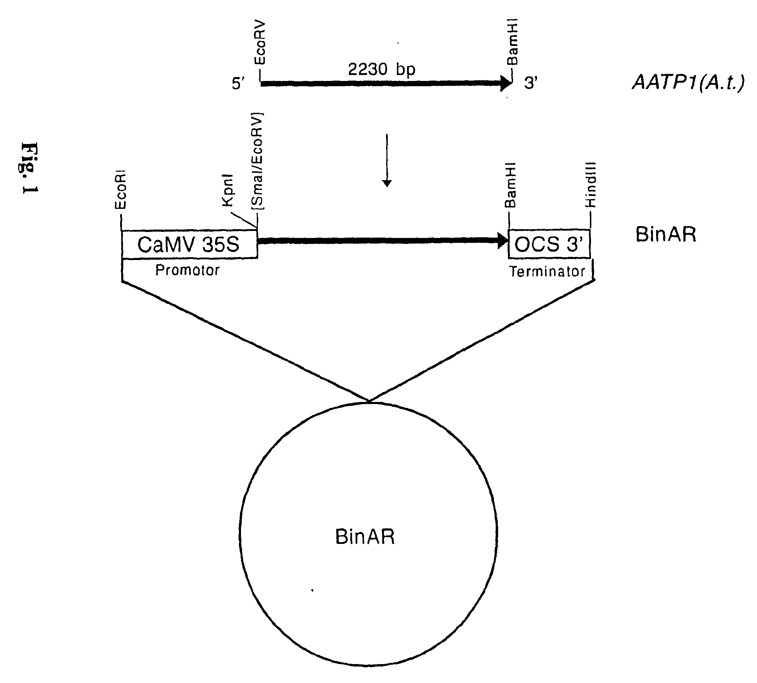

Zur Konstruktion eines Pflanzentransformationsvektors wurde ein 2230 bp langes EcoRV/BamHI-Fragment der AATP1-cDNA aus A. thaliana (Kampfenkel et al., FEBS Letters 374 (1995), 351-355) in den mit Smal/EcoRV und BamH1 geschnittenen Vektor pBinAR (Höfgen und Willmitzer, Plant Sci. 66 (1990), 221-230) ligiert. Durch die Insertion des cDNA-Fragmentes entsteht eine Expressionskassette (pJT31), die folgendermaßen aus den Fragmenten A, B und C aufgebaut ist (s. Fig. 1):
Das Fragment A (540 bp) enthält den 35S-Promotor aus dem Blumenkohlmosaikvirus.
Das Fragment B enthält neben den flankierenden Bereichen die proteincodierende Region eines ADP/ATP-Translokators aus A. thaliana (AATP1). Diese wurde wie oben beschrieben isoliert und in sense-Orientierung an den 35S-Promotor in pBinAR fusioniert.
Fragment C (215 bp) enthält das Polyadenylierungssignal des Octopin-Synthase Gens aus Agrobakterium tumefaciens.
Die Größe des Plasmids pJT31 beträgt ca. 14,2 kb.
Das Plasmid wurde mit Hilfe von Agrobakterien wie bei Rocha-Sosa et al. (EMBO J. 8 (1989), 23-29) in Kartoffelpflanzen transferiert. Als Ergebnis der Transformation zeigten transgene Kartoffelpflanzen eine Erhöhung der mRNA eines plastidären ADP/ATP-Translokators. Dies wurde mittels Northern Blot Analyse nachgewiesen (s.

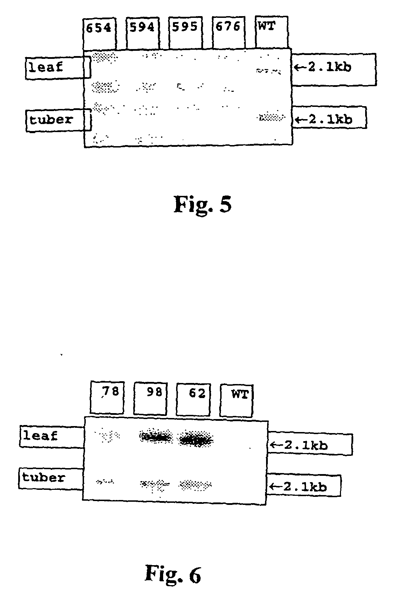

Fig. 6). Dazu wurde RNA nach Standardprotokollen aus Blatt- und Knollengewebe von Kartoffelpflanzen isoliert. 50µg RNA wurden auf einem Agarosegel aufgetrennt (1,5% Agarose, 1x MEN-Puffer, 16,6% Formaldehyd). Nach der Elektrophorese wurde die RNA mit 20x SSC mittels Kapillarblot auf eine Nylonmembran Hybond N (Amersham, UK) übertagen. Die RNA wurde auf der Membran duch UV-Bestrahlung fixiert. Die Membran wurde in Phosphathybridisierungspuffer (Sambrook et al., a.a.O) für 2h prähybridisiert und anschließend durch Zugabe der radioaktiv markierten Sonde für 10h hybridisiert.

### Beispiel 3

### Konstruktion des Plasmids pJT32 und Einführung des Plasmids in das Genom von Kartoffelpflanzen

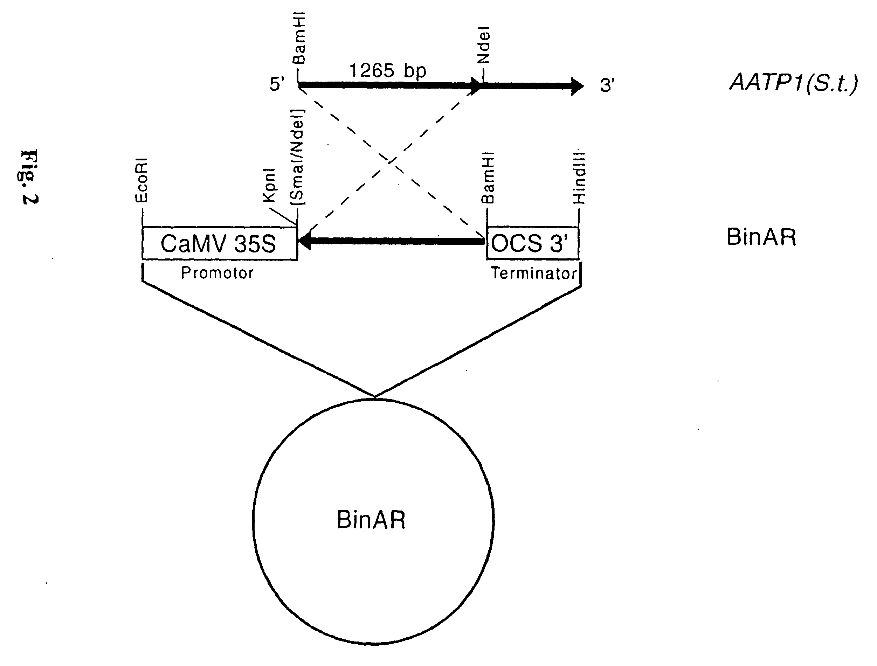

Zur Konstruktion eines Pflanzentransformationsvektors wurde ein 1265 bp langes BamHI/Ndel- Fragment der codierenden Region der AATP1-cDNA aus S. tuberosum (Genbank Y10821) in den mit Smal/Ndel und BamHI geschnittenen Vektor pBinAR (Höfgen und Willmitzer, Plant Sci. 66 (1990), 221-230) ligiert.
Durch die Insertion des cDNA-Fragmentes entsteht eine Expressionskassette, die folgendermaßen aus den Fragmenten A, B und C aufgebaut ist (s. Fig. 2):
Das Fragment A (540 bp) enthält den 35S-Promotor aus dem Blumenkohlmosaikvirus.
Das Fragment B enthält eine 1265 bp lange Region eines ADP/ATP-Translokators aus S. tuberosum (AATP1 S.t.). Diese wurde in antisense-Orientierung an den 35S-Promotor in pBinAR fusioniert.
Fragment C (215 bp) enthält das Polyadenylierungssignal des Octopin-Synthase Gens aus Agrobakterium tumefaciens.
Die Größe des Plasmids pJT32 beträgt ca. 13,3 kb.
Das Plasmid wurde mit Hilfe von Agrobakterien wie bei Rocha-Sosa et al. (EMBO J. 8 (1989), 23-29) in Kartoffelpflanzen transferiert.
Als Ergebnis der Transformation zeigten transgene Kartoffelpflanzen eine Verringerung der mRNA eines plastidären ADP/ATP-Translokators. Dies wurde mittels Northern Blot Analyse nachgewiesen (s. Fig. 5). Dazu wurde RNA nach Standardprotokollen aus Blatt- und Knollengewebe von Kartoffelpflanzen isoliert. 50µg RNA wurden auf einem Agarosegel aufgetrennt (1,5% Agarose, 1 x MEN-Puffer, 16,6% Formaldehyd). Nach der Elektrophorese wurde die RNA mit 20x SSC mittels Kapillarblot auf eine Nylonmembran Hybond N (Amersham, UK) übertagen. Die RNA wurde auf der Membran duch UV-Bestrahlung fixiert. Die Membran wurde in Phosphathybridisierungspuffer (Sambrook et al., a.a.O) für 2h prähybridisiert und anschließend durch Zugabe der radioaktiv markierten Sonde für 10h hybridisiert.

### Beispiel 4

### Analyse des Stärke-, Amylose- und des Zuckergehalts transgener Kartoffelpflanzen

Die Bestimmung des Gehaltes an löslichen Zuckern erfolgte wie bei Lowry und Passonneau in "A Flexible System of Enzymatic Analysis", Academic Press, New York, USA (1972) beschrieben. Die Bestimmung des Gehaltes an Stärke erfolgte wie bei Batz et al. (Plant Physiol. 100 (1992), 184-190) beschrieben.

**Tabelle 1:**

| Linie/Genotyp | Stärke in (µmolC6units/g Frischgewicht) | Lösliche Zucker in (µmol/g Frischgewicht) |
|---|---|---|
| Desiree/WT | 1094.0 | 26.49 |
| 654/antisense-AATP1 (S. tuberosum) | 574.2 | 42.52 |
| 594/antisense-AATP1 (S. tuberosum) | 630.2 | 48.76 |
| 595/antisense-AATP1 (S. tuberosum) | 531.4 | 45.92 |
| 676/antisense-AATP1 (S. tuberosum) | 883.0 | 40.60 |
| 62/ sense-AATP1(A. thaliana) | 1485.0 | 30.65 |
| 98/ sense-AATP1 (A. thaliana) | 1269.0 | 18.28 |
| 78/sense-AATP1 (A. thaliana) | 995.0 | 20.50 |

Die Bestimmung des Gehaltes an Amylose erfolgte wie bei Hovenkamp-Hermelink et al. (Potato Res. 31 (1988), 241-246) beschrieben:

| **Linie/Genotyp** | **% Amylose** |
|---|---|
| Desiree/WT | 18.8 |
| 654 /antisense-AATP1 (S. tuberosum) | 15.5 |
| 594/antisense-AATP1 (S. tuberosum) | 14.3 |
| 595/antisense-AATP1 (S. tuberosum) | 18.0 |
| 676/antisense-AATP1 (S. tuberosum) | 11.5 |
| 62/ sense-AATP1 (A. thaliana) | 27.0 |
| 98/ sense-AATP1 (A. thaliana) | 22.7 |
| 78/sense-AATP1 (A. thaliana) | 24.5 |

### Beispiel 5

### Herstellung einer Expressionskassette und Transformation von Rapspflanzen

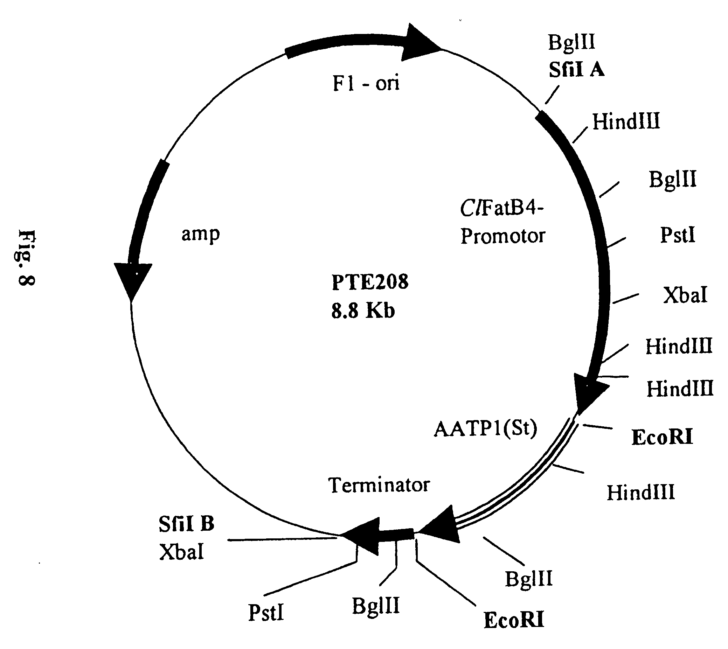

Die Expressionskassette pTE200 in einem pBluescript-Derivat (Short *et al.,* Nucl. Acid Res. 16, (1988), 7583-7600) trägt die Promotor- und Terminatorsequenzen des Thioesterase-Gens ClFatB4 (GenBank Accession: AJ131741) aus *Cuphea lanceolata* und geeignete Polylinkersequenzen zur Aufnahme verschiedener Nutzgene. Randständige Sfil-Erkennungsstellen mit nicht-kompatiblen Nukleotiden in der variablen Erkennungsregion erlauben einen gerichteten Transfer der gesamten Expressionskassette einschließlich des Nutzgens in die entsprechenden Restriktionsstellen des binären Plasmidvektors pMH000-0, einer Weiterentwicklung von pLH9000 (Hausmann und Töpfer, (1999): 9. Kapitel "Entwicklung von Plasmid-Vektoren" in Bioengineering für Rapssorten nach Maß, D. Brauer, G. Röbbelen und R. Töpfer (eds.) Vorträge für Pflanzenzüchtung Heft 45, 155-172), und verhindern beim aufnehmenden Vektor eine Rezirkularisierung der DNA.
Zur Herstellung der Expressionskassette pTE200 wurde zunächst aus dem genomischen Klon CITEg16 (WO95/07357), der das vollständige ClFatB4-Gen aus *C. lanceolata* trägt, ein ca. 3,3 kb lange promotortragende Sall-Bbvl Fragment isoliert. Hierzu wurde die Bbvl-Schnittstelle am 3'-Ende des Promotors geöffnet und derart modifiziert, daß das Fragment anschließend in den mit Sall und Smal geschnittenen pBluescript (Stratagene) aufgenommen werden konnte. Eine 1211 Nukleotide 5'-wärts liegende interne EcoRI-Schnittstelle des Fragments wurde deletiert, indem sie geöffnet, mittels T4 DNA-Polymerase modifiziert und anschließend wieder geschlossen wurde.
Die Terminatorsequenz wurde mittels der Polymerasekettenreaktion und spezifischen Oligonukleotidprimer an der CITEg16-Matrize (WO 95107357) amplifiziert und über die Primer mit einer Reihe von Polylinkerschnittstellen (MCS) versehen. Die Sequenzen der Primer lauten:
5'GAATTCCTGCAGCCCGGGGGATCCACTAGTCTCGAGAAGTGGCTGGGGGCC TTTCC3' (SEQ ID NO: 3)= 5'-primer: (MCS: EcoRI, PstI, SmaI, BamHI, SpeI Xhol; CIFatB4-Terminator: von Pos. 35 - 56) und
5'TCTAGAGGCCAAGGCGGCCGCTTCAACGGACTGCAGTGC3' (SEQ ID NO: 4) = 3'-primer: CIFatB4-Terminator: von Pos. 22 - 39, MCS: Notl, Styl, Sfil, Xbal. Das Amplifikat wurde mit EcoRI und NotI geschnitten und in die korrespondierenden Schnittstellen des pBlueSfi BA (Hausmann und Töpfer, s.o.) inseriert. Das promotortragende Fragment wurde mit BamHI geöffnet, modifiziert und anschließend mit Sall geschnitten, um es im pBlueSfi BA-Vektor über Sall und modifizierte Hindlll-Schnittstelle vor den Terminator zu plazieren. Es resultierte die Expressionskassette pTE200 (s. Figur 7).
Zur Konstruktiuon eines Pflanzentransformationsvektors wurde ein 2270 bp langes EcoRI-Fragment der AATP1 cDNA aus *Solanum tuberosum* (pTM1, Tjaden et al. The Plant Journal 16 (1998) 531-540) in den mit EcoR1 geöffneten Vektor pTE200 ligiert. Die Orientierung wurde mittels Restriktionsverdau überprüft. Es resultiert das Plasmid pTE208 (Figur 8). In einem anschließenden Schritt wurde das Sfil-Fragment aus pTE208 in die Polylinkerschnittstellen des binären Vektors pMH000-0 (Figur 9) gerichtet inseriert. Es resultierte der Vektor pMH 0208.
Der binäre Plasmidvektor pMH000-0 ist eine Weiterentwicklung von pLH9000 (Hausmann und Töpfer, s.o.) mit alternativen Selektionsmarkern für die Pflanzentransformation. Hierzu wurde das Sulfonamidgen (sul) zusammen mit der Signalpeptidsequenz (tp) für plastidären Import der kleinen Untereinheit der Ribulosebisphosphat Carboxylase aus dem Vorläuferplasmid von pS001 (Reiss et al., Proc. Natl. Acad. Sci. USA 93, (1996), 3094-3098) nach Modifizierung der Asp718- zur Xhol-Schnittstelle isoliert. Das Xhol-Sall Fragment wurde in die XhoI- und BamHI-Schnittstellen eines pBluescript-Derivat vor den Terminator des Nopalinsynthasegens (pAnos) nach Modifizierung von Sall und BamHI eingeführt. In einer anschließenden Dreifragmentligation wurden das resultierende tpsul-pAnos Fragment (Xhol - Xbal) und das Xhol - HindIII Fragment aus pRT103pat (Töpfer *et al*., Methods in Enzymol. 217, (1993), 66-78) mit dem mit HindIII und Xbal geöffneten Plasmid pK18 (Pridmore, Gene 56, (1987), 309-312) vereint. Dabei kam das Gen für die Phosphinotricin Acetyltransferase mit dem Terminator des CaMV35S rna-Gens aus pRT103pat in entgegengesetzter Orientierung zur tpsul-pAnos-Einheit zu liegen. Zur Vervollständigung dieser doppelten Selektionseinheit (für Resistenz gegen das Herbizid Basta und das Sulfonamid Sulfadiazin) wurde ein dualer Promotor des CaMV35S rna-Gens als Xhol-Fragment aus einem Abkömmling von pROA93 (Ott *et al.,* Mol. Gen. Genet. 221, (1990), 121-124) in die Xhol-Schnittstelle zwischen die Resistenz-vermittelnden Gensequenzen inseriert. Die resultierende duale Selektionskassette wurde über Xbal und HindIII gegen die Kanamycinkassette im pLH9000-Vorläuferplasmid (Hausmann und Töpfer, s.o.) nach entsprechenden Modifikationen im angrenzenden Polylinker ausgetauscht. Daraus resultierte der binäre Plasmidvektor pMH000-0.

Die Transformation von Hypokotylexplantaten von Raps der Sorte Drakkar erfolgte nach dem Protokoll von De Block (Plant Physiol. 91 (1989), 694-701) mit Hilfe von Agrobakterien (Stamm GV 3101 C58C1 Rifr), die den binären Vektor pMH0208 (ATP/ADP Transporter sense) trugen. Sprosse wurden auf selektivem Nährmedium (Sulfonamid) regeneriert und im Gewächshaus bis zur Samenreife kultiviert. Mittels PCR und Blatttest (Toleranz gegenüber Glufosinatammonium (Basta® )) wurde überprüft, welche Pflanzen das Transgen enthielten. Von diesen Pflanzen wurden heranreifende Embryonen verschiedener Entwicklungsstadien geerntet und in flüssigem Stickstoff gelagert.
Zur Bestimmung des Ölgehalts wurde reifes Saatgut von transgenen Rapslinien und von Kontrollinien mit Hilfe der nicht-invasiven Nahinfrarotspektroskopie (beispielsweise beschrieben bei Schutz et al., J. Near Infrared Spectrosc. 6, (1998), A125-A130; Starr et al., J. Agric. Sci. 104(2), (1985), 317-323) untersucht.

## Patentansprüche

1. Transgene Pflanzenzelle, die genetisch modifiziert ist, wobei die genetische Modifikation in der Einführung eines fremden Nucleinsäuremoleküls besteht, das einen plastidären ADP/ATP-Translokator codiert und dessen Expression zur Erhöhung der plastidären ADP/ATP-Translokatoraktivität führt im Vergleich zu entsprechenden nicht genetisch modifizierten Pflanzenzellen aus Wildtyp-Pflanzen, und wobei die transgene Pflanzenzelle einen erhöhten Stärkegehalt im Vergleich zu entsprechenden nicht genetisch modifizierten Pflanzenzellen aufweist und/oder eine Stärke synthetisiert, die im Vergleich zu Stärke aus entsprechenden nicht genetisch modifizierten Pflanzenzellen einen erhöhten Amylosegehalt aufweist.

2. Transgene Pflanzenzelle nach Anspruch 1, wobei das Nucleinsäuremolekül einen plastidären ADP/ATP-Translokator aus Arabidopsis thaliana codiert.

3. Transgene Pflanze enthaltend transgene Pflanzenzellen nach Anspruch 1 oder 2.

4. Transgene Pflanze nach Anspruch 3, die eine stärkespeichernde Pflanze ist.

5. Transgene Pflanze nach Anspruch 4, die eine Mais-, Weizen- oder Kartoffelpflanze ist.

6. Verfahren zur Herstellung einer transgenen Pflanze, die im Vergleich zu Wildtyp-Pflanzen einen erhöhten Stärkegehalt aufweist und/oder deren Stärke einen erhöhten Amylosegehalt im Vergleich zu Stärke aus Wildtyp-Pflanzen aufweist, wobei
(a) eine pflanzliche Zelle genetisch modifiziert wird durch die Einführung eines fremden Nucleinsäuremoleküls, das einen plastidären ADP/ATP-Translokator codiert und dessen Expression zur Erhöhung der plastidären ADP/ATP-Translokatoraktivität in der Zelle führt;
(b) aus der gemäß Schritt (a) hergestellten Zelle eine Pflanze regeneriert wird; und
(c) aus der gemäß Schritt (b) erzeugten Pflanze gegebenenfalls weitere Pflanzen erzeugt werden.

7. Transgene Pflanze erhältlich durch das Verfahren nach Anspruch 6.

8. Vermehrungsmaterial von Pflanzen nach einem der Ansprüche 3 bis 5 oder 7, wobei dieses Vermehrungsmaterial transgene Zellen nach Anspruch 1 oder 2 enthält.

9. Verwendung von Nucleinsäuremolekülen, die einen plastidären ADP/ATP-Translokator codieren, zur Herstellung von transgenen Pflanzen, die im Vergleich zu Wildtyp-Pflanzen einen erhöhten Stärkegehalt aufweisen und/oder eine Stärke synthetisieren, die im Vergleich zu Stärke aus Wildtyp-Pflanzen einen erhöhten Amylosegehalt aufweisen.

10. Verfahren zur Herstellung einer modifizierten Stärke umfassend die Extraktion der Stärke aus einer Pflanze nach einem der Ansprüche 3 bis 5 oder aus Vermehrungsmaterial nach Anspruch 8.

## Claims

1. Genetically modified transgenic plant cell, wherein the genetic modification consists in the introduction of a foreign nucleic acid molecule encoding a plastidic ADP/ATP translocator, the expression of which leads to an increase in plastidic ADP/ATP translocator activity compared to corresponding genetically unmodified plant cells from wild-type plants, and wherein the transgenic plant cell has an increased starch content compared to corresponding genetically unmodified plant cells and/or synthesizes a starch having an increased amylose content compared to starch from corresponding genetically unmodified plant cells.

2. Transgenic plant cell according to claim 1, wherein the nucleic acid molecule encodes a plastidic ADP/ATP translocator from Arabidopsis thaliana.

3. Transgenic plant containing transgenic plant cells according to claim 1 or 2.

4. Transgenic plant according to claim 3 being a starch-storing plant.

5. Transgenic plant according to claim 4, being a maize, wheat or potato plant.

6. Process for the production of a transgenic plant having an increased starch content compared to wild-type plants and/or the starch of which has an increased amylose content compared to starch from wild-type plants, wherein
(a) a plant cell is genetically modified by the introduction of a foreign nucleic acid molecule encoding a plastidic ADP/ATP translocator, the expression of which leads to an increase of plastidic ADP/ATP translocator activity in the cell;
(b) a plant is regenerated from the cell produced according to step (a); and
(c) optionally further plants are produced from the plant produced according to step (b).

7. Transgenic plant obtainable by the process according to claim 6.

8. Propagation material of plants according to any one of claims 3 to 5 or 7, wherein this propagation material contains transgenic cells according to claim 1 or 2.

9. Use of nucleic acid molecules encoding a plastidic ADP/ATP translocator for the production of transgenic plants having an increased starch content compared to wild-type plants and/or synthesizing a starch having an increased amylose content compared to starch from wild-type plants.

10. Process for the production of a modified starch comprising the extraction of the starch from a plant according to any one of claims 3 to 5 or from propagation material according to claim 8.

## Revendications

1. Cellule de plante transgénique, qui est génétiquement modifiée, la modification génétique consiste en l'introduction d'une molécule d'acide nucléique étrangère, qui code un translocateur ADP/ATP plastidique et dont l'expression mène à l'augmentation de l'activité du translocateur ADP/ATP plastidique par rapport aux cellules de plante correspondantes génétiquement non modifiées à partir de plantes de type sauvage, et la cellule de plante transgénique présente une teneur en amidon augmentée par rapport aux cellules de plante correspondantes génétiquement non modifiées et/ou synthétise un amidon qui présente une teneur en amylose augmentée par rapport à l'amidon à partir de cellules correspondantes de plante génétiquement non modifiées.

2. Cellule de plante transgénique selon la revendication 1, où la molécule d'acide nucléique code un translocateur ADP/ATP plastidique *d'Arabidopsis thaliana.*

3. Plante transgénique renfermant des cellules de plante transgénique selon la revendication 1 ou 2.

4. Plante transgénique selon la revendication 3, qui est une plante de stockage de l'amidon.

5. Plante transgénique selon la revendication 4, qui est une plante de maïs, de blé ou de pomme de terre.

6. Procédé pour la production d'une plante transgénique qui présente une teneur en amidon augmentée par rapport aux plantes de type sauvage, et/ou son amidon présente une teneur en amylose augmentée par rapport à l'amidon de plantes de type sauvage, où
(a) une cellule de plante est génétiquement modifiée par l'introduction d'une molécule d'acide nucléique étrangère, qui code un translocateur ADP/ATP plastidique et dont l'expression mène à l'augmentation de l'activité du translocateur ADP/ATP plastidique dans la cellule ;
(b) une plante est régénérée à partir de la cellule préparée selon l'étape (a) ; et
(c) éventuellement d'autres plantes sont produites à partir de la plante produite selon l'étape (b)..

7. Plante transgénique que l'on peut obtenir l'aide du procédé selon la revendication 6.

8. Matière de propagation de plantes selon l'une des revendications 3 à 5 ou 7, où cette matière de propagation renferme des cellules transgéniques selon la revendication 1 ou 2.

9. Utilisation de molécules d'acide nucléique qui codent un translocateurs ADP/ATP plastidique, pour la préparation de plantes transgéniques qui présentent une teneur en amidon augmentée par rapport aux plantes de type sauvage et/ou synthétisent un amidon qui présente une teneur en amylose augmentée par rapport aux plantes de type sauvage.

10. Procédé pour la préparation d'un amidon modifié comprenant l'extraction de l'amidon à partir d'une plante selon l'une des revendications 3 à 5 ou d'une matière de propagation selon la revendication 8.
